# EUROPEAN PATENT SPECIFICATION

(11) **EP 2 542 207 B1**
(45) Date of publication and mention of the grant of the patent: **03.05.2017**
(21) Application number: 11705632.5
(22) Date of filing: 02.03.2011
(51) Int. Cl.: A61K 8/04, A61K 9/12, A61K 9/00

(54) **FOAM FORMULATIONS CONTAINING AT LEAST ONE TRITERPENOID**
SCHAUMFORMULIERUNGEN ENTHALTEND MINDESTENS EIN TRITERPENOID
FORMULATIONS DE MOUSSE CONTENANT AU MOINS UN TRITERPÉNOÏDE

(30) Priority: 02.03.2010 EP 10155214; 02.03.2010 US 309706 P
(43) Date of publication of application: 09.01.2013
(73) Proprietor: Neubourg Skin Care GmbH & Co. KG, 48268 Greven (DE)
(72) Inventor: NEUBOURG, Thomas, 59368 Werne (DE)
(74) Representative: Jungblut & Seuss
(86) International application number: PCT/EP2011/053131
(87) International publication number: WO 2011/107522

(56) References cited:
- WO-A1-03/026603
- WO-A1-2010/060896
- US-A1- 2006 057 075
- US-A1- 2007 231 418
- DANIELS R ET AL: "Skin care: Betulin for surfactant-free emulsions", PHARMAZEUTISCHE ZEITUNG 20080313 DE, vol. 153, no. 11, 13 March 2008 (2008-03-13), pages 34-35, XP008124996, ISSN: 0031-7136

## Description

### Technical field of the invention

The present invention relates to cosmetic and dermatological foam formulations, particularly to foam creams, based on emulsions, particularly of the oil-in-water type, wherein the emulsion comprises at least one triterpenoid.

### Background of the invention

### 1. Triterpenoids

Terpenes are hydrocarbons of natural origin having carbon skeletons, which are formally derived from isoprene. They can be subdivided into hemiterpenes (C5-skeleton), monoterpenes (C10-skeleton), sesquiterpenes (C15-skeleton), diterpenes (C20-skeleton), sesterterpenes (C25-skeleton), triterpenes (C30-skeleton) and tetraterpenes (C40-skeleton).

In contrast to that terpenoids are natural products and substances derived therefrom, which may also contain oxygen-containing functional groups. They are classified in the same manner as for terpenes by the number of carbon atoms of the underlying skeleton. Triterpenoids are thus based on a C30-skeleton.

Triterpenoids often possess a tetracyclic or pentacyclic structure. Pentacylic structures consisting of five annulated six-membered rings comprise for example the oleanane structure, the friedelane structure and the ursane structure. The pentacyclic structure of the lupane-type however contains a five-membered ring in addition to four six-membered rings.

Exemplary triterpenoids having an oleanane structure are oleanolic acid, erythrodiol, β-amyrin, glycyrrhetinic acid, and α-boswellic acid. Exemplary triterpenoids having an ursane structure are ursolic acid, α-amyrin, β-boswellic acid and corosolic acid. An exemplary triterpenoid having a friedelane structure is 2α,3β-friedelanediol. For example, the triterpenoids betulin, betulinic acid, betulinic acid methyl ester, betulin aldehyde, betulonic acid, betulon aldehyde and lupeol possess a lupane structure.

Triterpenoids can be of natural or synthetic origin. Typically they are obtained by extraction from plant material. Extraction processes for the isolation of triterpenoids are described e.g. in the international applications WO 01/72315 A1 and WO 2004/016336 A1,

Birch cork is the tissue in the flora containing the highest amounts of triterpenoids (Hayek et al.; A bicentennial of Betulin, Phytochemistry 1989; 28: 2229-42). Triterpenoids are also prevalent in other plants in smaller amounts. For example, betulinic acid can be found in the bark of the sycamore tree (Galgon T. et al.; Phytochemical Analysis 1999; 10: 187-90.). Betulinic acid and oleanolic acid are contained in the mistletoe (Jäger S et al., Planta Med 2007; 73:157-62), as well as in rosemary (Abe F et al., Biol Pharm Bull 2002; 25: 1485-7), which also contains ursolic acid.

Means are also known to further process triterpenoid mixtures isolated as natural products by chemical reactions, e.g. in order to effect an accumulation of certain triterpenoids. For example, WO 02/085921 A2 describes a process for the manufacture of a pure boswellic acid from a boswellic acid mixture.

Triterpenoids possess various pharmacological properties. For example, for triterpenoids such as e.g. betulin, betulinic acid or oleanolic acid, antibacterial, hepatoprotective, antiplasmodial, antiviral, antitumor, wound healing promoting and anti-inflammatory properties were described.

Also for boswellic acids, which are ingredients of the resin of the olibanum tree Boswellia, and of the myrrh tree Commiphora, anti-inflammatory, cytostatic and antitumor properties were described.

The pharmacological properties of the triterpenoids make them interesting inter alia also for dermatological and cosmetic applications. For example, a pilot study for the local treatment of actinic keratoses with a cream containing a birch cork extract demonstrated the good efficacy and compatibility of the latter (Huyke C. et al., Journal der Deutschen Dermatologischen Gesellschaft, 2006, 4 (2), pages 132-136). Furthermore, WO 2005/123037 A1 discloses oleogels for cosmetic-pharmaceutical applications, containing a non-polar liquid and a triterpenoid-containing oleogel-forming agent. According to the disclosure of WO 2005/123037 A1, the advantage of the oleogel lies in the simplicity of its formulation, wherein the triterpene functions simultaneously as a pharmaceutically active substance and as a gel-forming agent.

### 2. Emulsions

Emulsions represent an important product type in the field of cosmetic and/or dermatological preparations, which is used in different areas of application. Generally, the term emulsion relates to heterogeneous systems consisting of two liquids that are not miscible with each other or only miscible to a limited extent, which are typically designated as phases. In an emulsion, one of the two liquids is dispersed in the other liquid in the form of minute droplets. In case that the two liquids are water and oil, and the oil droplets are finely dispersed in the water, the emulsion is an oil-in-water emulsion (O/W-emulsion, e.g. milk). The basic character of an O/W-emulsion is defined by the water. In case of a water-in-oil emulsion (W/O-emulsion, e.g. butter), the opposite principle applies, wherein the basic character is in this case defined by the oil.

In order to achieve the durable dispersion of a liquid in another liquid, emulsions in a conventional sense require the addition of a surface active agent (emulsifier). Emulsifiers have an amphiphilic molecular structure consisting of a polar (hydrophilic) and a non-polar (lipophilic) part of the molecule, which are spatially separated from each other. In simple emulsions, one of the phases contains finely dispersed droplets of the second phase, which are enclosed by an emulsifier shell (water droplets in W/O-emulsions or lipid vesicles in O/W-emulsions). Emulsifiers reduce the surface tension between the phases by being arranged at the interface between the two liquids. They form interfacial films at the oil/water phase interface which countervails the irreversible coalescence of the droplets. Mixtures of emulsifiers are often used for stabilizing emulsions.

The term "emulsifier" or "conventional emulsifier" is known in the art. Conventional emulsifiers and their use are described, e.g., in the publications: Pflegekosmetik, 4th edition, Wissenschaftliche Verlagsgesellschaft mbH Stuttgart, pages 151 to 159, and Fiedler Lexikon der Hilfsstoffe, 5th edition, Editio Cantor Verlag Aulendorf, pages 97 to 121.

The IUPAC defines the term "emulsifier" as follows: "Emulsifiers are surface active substances. They are preferably arranged in the interface between oil phase and aqueous phase, and thereby reduce the surface tension. Even in low concentration, emulsifiers facilitate the formation of an emulsion. Moreover, these substances are able to enhance the stability of emulsions by reducing the rate of aggregation and/or coalescence."

Conventional emulsifiers can be classified, based on their hydrophilic part of the molecule, into ionic (anionic, cationic and amphoteric) emulsifiers and non-ionic emulsifiers:
- The probably best known example of an anionic emulsifier is soap which is the conventional name for water-soluble sodium or potassium salts of saturated and unsaturated higher fatty acids.
- Important representatives of cationic emulsifiers are quaternary ammonium compounds.
- The hydrophilic part of the molecule of non-ionic emulsifiers often consists of glycerol, polyglycerol, sorbitans, carbohydrates or polyoxyethylene glycols, and is mostly connected by means of ester and ether bonds to the lipophilic part of the molecule. The latter typically consists of fatty alcohols, fatty acids or iso-fatty acids.

By variation of the structure and the size of the polar and the non-polar part of the molecule, lipophilicity and hydrophilicity of emulsifiers may be modified to a large extent.

The correct choice of the emulsifiers is decisive for the stability of an emulsion. In this respect, the characteristics of all substances contained in the system need to be considered. In case of skin care emulsions, for example, polar oil components such as e.g. UV filters may cause instabilities. Therefore, in addition to emulsifiers, other stabilizers are additionally used, which for example increase the viscosity of the emulsion and/or act as protective colloid.

### 3. Emulsifier-free emulsions

Since decades, conventional emulsifiers form the basis for the development of skin care preparations. Emulsifiers were used as adjuvants for the manufacture of and especially for stabilizing emulsions. Recently it was indicated that the use of emulsifiers in skin care preparations may cause problems e.g. in the case of sensitive skin, since emulsifiers typically disturb the integrity of the natural skin barrier, which may, when cleaning the skin, result in a loss of natural barrier substances of the skin. The loss of natural barrier substances may cause an increased roughness, dry skin, cracked skin, and wear eczema.

Furthermore, the use of emulsifiers may result in the conversion of lamellar structures of the lipid barrier into vesicular structures, such as e.g. micelles or mixed micelles. These vesicles "destroy" at least a part of the barrier layer of the skin, and thereby locally increase the permeability of the barrier layer membrane. Due to this opening of the barrier layer of the skin, the transepidermal water loss (TEWL) is at least temporarily increased, and simultaneously, the capacity of the skin to bind moisture is decreased. A continuous application of skin care preparations containing conventional emulsifiers may even result in the skin being no longer able to maintain its protective function.

Since conventional emulsifiers are repeatedly cited as the cause of incompatibilities of skin care products, such as e.g. a dysfunction of the skin barrier or Mallorca acne, the cosmetics industry is looking for alternatives to the conventional formulations in the form of emulsifier-free emulsions.

Emulsifier-free emulsions are free of emulsifiers in a conventional sense, i.e. amphiphilic substances having a low molecular weight (i.e. molecular weight < 5000 g/mol), that, in suitable concentrations, can form micelles and/or other liquid crystalline aggregates.

The term "emulsifier-free" is established in the art. According to a definition of the Society for Dermopharmacy, which was adopted by an interdisciplinary consent among pharmacists, dermatologists and other experts (http://www.dermotopics.de/german/ausgabe_1_03_d/emulgatorfrei_1_2003_d.htm), a formulation can be designated as "emulsifier-free" when it is stabilized by means of surface active macromolecules (molecular weight of above 5000 g/mol), instead of by emulsifiers in a narrower sense (i.e. conventional emulsifiers).

For example solid- or polymer-stabilized emulsions have proved to be a promising approach for emulsifier-free emulsions with the aim of obtaining sufficiently stable and cosmetically attractive products, which help to avoid the disadvantages connected with conventional emulsifiers.

### 4. Solid-stabilized emulsions

An example of emulsifier-free emulsions are emulsions stabilized by solids. Solid-stabilized emulsions, which are also designated in the art as Pickering emulsions, are stabilized by means of finely dispersed solid particles and, as far as possible, allow for the abdication of conventional emulsifiers.

In solid-stabilized emulsions, the solid substance accumulates at the oil/water phase interface in the form of a layer whereby the coalescence of the dispersed phase is prevented.

Suitable solid emulsifiers are in particular particulate, inorganic or organic solids, which are wettable by both hydrophilic and lipophilic liquids. Preferably, in the solid-stabilized emulsions or Pickering emulsions, e.g. titanium dioxide, zinc oxide, silicon dioxide, Fe₂O₃, veegum, bentonite or ethyl cellulose are used as solid emulsifiers.

In EP 1 352 639 A1 or DE 101 62 840, Pickering emulsions are presented which are used in form of lotions, creams or gels. WO 2004/017930 describes further Pickering emulsions.

### 5. Polymer-stabilized emulsions

A further example of emulsifier-free emulsions are polymer-stabilized emulsions. In the case of polymer-stabilized emulsions, and in contrast to the conventional emulsions, the required stabilization is not achieved by amphiphilic, surfactant-like emulsifiers, but by means of suitable macromolecules. The irritation potential of formulations that are stabilized in this way differs significantly from that of emulsions which are stabilized by conventional emulsifiers. Due to their high molecular weight, polymeric emulsifiers cannot penetrate into the stratum corneum. Therefore, undesired interactions, e.g. in the sense of Mallorca acne, are not to be expected.

Polymer-stabilized emulsions, in particular the underlying type of stabilization, as well as suitable polymeric emulsifiers are disclosed in the European patent application having the application number 09 015 330.5 and the title "Emulsifier-free, polymer-stabilized foam formulations" of the applicant Neubourg Skin Care GmbH & Co. KG, in the chapter "Polymer-stabilized emulsions". The content of the European patent application 09 015 330.5, in particular of the chapter regarding Polymer-stabilized emulsions (pages 6 to 10) is hereby incorporated by reference.

### 6. DMS-containing emulsions

Today, cream bases are often used which employ a variety of natural or skin-like ingredients promising a better skin compatibility especially in case of sensitive skin. It has been shown that by using skin-like ingredients an improved skin care can be achieved. In these cream bases for example several components of the natural skin lipids are replaced, such as e.g. triglycerides by (vegetable) caprylic acid/capric acid triglycerides, squalene by (vegetable) squalane, ceramides by ceramide 3 (of yeast origin), cholesterol by (vegetable) phytosterols, and phospholipids by (vegetable) phospholipids.

In this concept, various typical additives such as fragrances, colorants, comedogenic lipids (e.g. mineral oils), preservatives and physiologically critical emulsifiers are preferably omitted, since these substances are potentially sensitizing and may cause skin irritations. In particular, these formulations are preferably prepared without conventional emulsifiers in order to avoid the above-mentioned disadvantages thereof.

Systems reverting to specifically composed membrane lipids which exhibit a lamellar structure of the membrane and thus simulate the structure of the physiological skin-lipid-barrier, are particularly known in the art under the designation "DMS^{®}" (Derma Membrane Structure). A commercial product having a DMS^{®} basis which is characterized as being "emulsifier-free" is for example marketed under the name Physiogel^{®} cream.

### 7. Triterpenoid-containing emulsions

WO 01/72315 A1 discloses emulsions, wherein the aqcous and fatty phase are emulsified by a plant extract, wherein the plant extract comprises at least one triterpenoid and/or at least one derivative of a triterpenoid. The triterpenoid and/or its derivative are stated to preserve and emulsify the emulsion and further to be pharmaceutically active. According to the disclosure of WO 01/72315 A1 it is thereby achieved that the emulsion does not contain any additional preservatives, whereby it possesses a particularly high degree of purity and a good compatibility, particularly in the case of problematic applications on damaged skin.

### 8. Foam formulations

A particular application form of cosmetic and/or dermatological emulsions is the application as foams. Foam formulations have the advantage that they can be easily distributed on the skin. The foamy consistency is experienced as comfortable and the products normally leave a good skin feeling. In particular, the physical structure of the foam also acts positively on the protective function of the skin. Foams are complicated physical structures that require a particular adjustment of the components constituting the foam. In general, foams are obtained by spraying an emulsion formulation or an aqueous surfactant (stabilizer) solution. For example, the emulsion charged with a propellant is dispensed from a pressurized container (in the literature and the patent literature such systems are also called aerosol foams). The pressurized mixture of emulsion and propellant expands and forms the foam bubbles. In particular, the dispersed oil phase, in which the oil-soluble gas is dissolved, expands. However, foams can also be formed by means of other systems such as, for example, pump sprays.

Upon application, balanced foam formulations have a stable two-phase or multiphase, polydisperse structure that forms a network structure on the skin which is comparable to a membrane. Such network structures have the advantage that they develop a protective action, for example against contact with water, however, allow for the unhindered gas exchange with the environment. In such foams, there is practically no disturbance of the *perspiratio insensibiles*, and no corresponding heat build-up. Thereby, the positive properties of a protective and nurturing action are combined with an unchanged perspiration.

Foam formulations often contain conventional emulsifiers that serve for the stabilization of the emulsion and for the subsequent foam stability. However, as discussed before, the latter are repeatedly cited as the cause of skin incompatibilities. Nonetheless, the addition of suitable stabilizers cannot be completely omitted, because disperse systems, as already described, for example emulsions, are thermodynamically instable.

In WO 2008/138894 foam formulations on the basis of emulsifier-free Pickering emulsions are described.

WO 2008/155389 describes foam formulations on the basis of emulsions, the oil phase of which comprises at least one membrane-forming substance forming lamellar arranged membranes in the foam formulation, wherein the emulsions are preferably emulsifier-free.

However, there remains a need for skin care compositions which satisfy the dermal requirements and thus allow for an optimum skin protection and an optimum skin care.

### Summary of the invention

The applicant has now discovered, that O/W emulsions containing at least one triterpenoid selected from the group consisting of betulin, betulinic acid, betulinic acid methyl ester, betulin aldehyde, betulonic acid, betulon aldehyde, lupeol, (C1-C6) alkyl esters of the aforementioned acids, or mixtures thereof, are a suitable basis for foam formulations. Thereby, the positive characteristics of foam formulations are combined with those of triterpenoid-containing cosmetics. In particular, foam formulations without conventional emulsifiers or with very low contents of conventional emulsifiers can be produced, that combine the positive characteristics of the foam, namely the physical structure and the pleasant applicability, with a good skin compatibility and, if applicable, with a nurturing or healing effect. This makes such foam formulations particularly usable for cosmetic and dermatological formulations for sensitive skin types. Compatibility and user friendliness are advantageously combined.

It is not self-evident that the foaming of triterpenoid-containing emulsions results in stable foam products. Forms are obtained, as already mentioned, by incorporating (pressure) liquefied propellants into O/W-emulsion systems or upon dispensing of the formulation/emulsion without propellant from a container other than a pressurized container. When the propellant dissolved in the dispersed oil phase evaporates upon foaming, a foam (gas-in-liquid dispersion) is formed. The evaporation or expansion of the propellant dissolved in the dispersed oil phase leads to a dilatation of the dispersed oil phase. It has now been surprisingly found that upon foaming of the emulsions underlying the foam formulations according to the invention, no breaking of the formulation occurs and a foam is formed that is suitable for use in pharmaceutical and cosmetic products. The latter is stable enough in order to be, e.g. applied to the skin.

In particular it has been surprisingly found that emulsions which contain in addition to the at least one triterpenoid still further, in particular emulsion-stabilizing ingredients, such as membrane-forming substances, emulsifier-copolymers, and/or solid emulsifiers, provide particularly suitable bases for foam formulations. For example, an emulsion comprising an oil phase and an aqueous phase, which comprises at least one triterpenoid, selected from the group consisting of betulin, betulinic acid, betulinic acid methyl ester, betulin aldehyde, betulonic acid, betulon aldehyde, lupeol, (C1-C6) alkyl esters of the aforementioned acids, or mixtures thereof, wherein the oil phase comprises at least one membrane-forming substance forming a lamellar arranged membrane in the foam formulation, provides a basis for foam formulations which results in particularly compact and creamy foams.

Further the applicant has discovered, that an O/W emulsion comprising an oil phase and an aqueous phase, wherein the emulsion comprises at least one triterpenoid selected from the group consisting of betulin, betulinic acid, lupeol, (C₁-C₆) allyl esters of the aforementioned acids, or mixtures thereof, and wherein the oil phase comprises at least one membrane-forming substance forming a lamellar arranged membrane in the emulsion, is a particularly suitable composition for cosmetic or pharmaceutical products, and in particular provides a particularly suitable basis for foam formulations.

The invention thus relates to foam formulations comprising an O/W emulsion, comprising an oil phase and an aqueous phase, wherein the emulsion comprises at least one triterpenoid. Preferably the foam formulations are a foam cream.

Further the invention relates to a claimed O/W emulsion comprising an oil phase and an aqueous phase, wherein the emulsion comprises at least one triterpenoid selected from the group consisting of betulin, betulinic acid, lupeol, C₁-C₆) alkyl esters of the aforementioned acids, or mixtures thereof, and wherein the oil phase comprises at least one membrane-forming substance forming a lamellar arranged membrane in the emulsion.

Further the invention relates to the use of the foam formulations according to the invention as carrier for an active agent, as skin care agent, as skin cleaning agent, as sunscreen, or for the manufacture of a cosmetic, a medical product or a medicament. The invention also relates to the use of the emulsion according to the invention as carrier for an active agent, as skin care agent, as skin cleaning agent, as sunscreen, or for the manufacture of a cosmetic, a medical product or a medicament.

Further the invention relates to the use of an emulsion, comprising an oil phase and an aqueous phase, wherein the emulsion comprises at least one triterpenoid, for the manufacture of a foam formulation.

Further the invention relates to the use of at least one triterpenoid for the stabilization of foam formulations comprising an O/W emulsion.

### Detailed description of the invention

### 1. Definitions

According to the present invention, foam formulations are formulations, in particular O/W emulsions, that are evidently adapted for the formation of a foam. In particular, the formulations may be filled, either together with a (pressure) liquefied propellant into a pressurized container, or without propellant into a container other than a pressurized container that allows for the formation of a foam upon dispensing of the formulation/emulsion. For example, pump spray containers may be used. According to an aspect of the invention conventional emulsifiers are amphiphilic substances (having a hydrophilic and a lipophilic part of the molecule, which are spatially separated from each other) having a molecular weight < 5000 g/mol, which form interfacial films at the oil/water phase interface.

According to a further aspect of the invention conventional emulsifiers are amphiphilic substances having a molecular weight < 5000 g/mol, which, in suitable concentrations, can form micelles and/or other liquid crystalline aggregates. Compounds or mixtures of substances which result instead of micelle formation in the formation of a lamellar arranged membrane in the sense of the present invention are however no conventional emulsifiers according to the present invention.

According to a further aspect of the invention conventional emulsifiers are all surface active substances that are present in the emulsion neither as solid nor as polymer, in particular under conventional storage and application temperatures, such as e.g. room temperature.

This means, that e.g. fatty alcohols with a chain length of 10 to 40 carbon atoms fulfill the definition of a conventional emulsifier according to the invention, as far as they are present in an emulsion, due to the formulation/composition thereof, not as a solid, but for example in liquid crystalline or dissolved form. In contrast, if the fatty alcohols with a chain length of 10 to 40 carbon atoms are present in the emulsion as a solid, they do not fulfill the definition of a conventional emulsifier according to the invention.

According to yet a further aspect of the invention, conventional emulsifiers according to the present invention are anionic, cationic, amphoteric and non-ionic surfactants. Typical representatives of anionic surfactants are neutralized branched and/or unbranched, saturated or unsaturated fatty acids having a chain length of 10 to 40 carbon atoms. Typical representatives of cationic surfactants are ammonium compounds. Typical representatives of non-ionic surfactants have a hydrophilic part of the molecule, such as glycerol, polyglycerol, sorbitan, carbohydrates or polyoxyethylene glycols, that is connected by means of ester and/or ether bonds to the lipophilic part of the molecule which typically consists of fatty alcohols, fatty acids or iso-fatty acids. For example, polyethoxylated fatty acid esters having a chain length of 10 to 40 carbon atoms and a degree of ethoxylation of 5 to 100 belong to this group. Furthermore, saturated and/or unsaturated, branched and/or unbranched fatty alcohols having a chain length of 10 to 40 carbon atoms belong to the group of non-ionic emulsifiers. Conventional emulsifiers are often used in combinations.

Triterpenoids selected from the group consisting of betulin, betulinic acid, betulinic acid methyl ester, betulin aldehyde, betulonic acid, betulon aldehyde, lupeol, (C1-C6) alkyl esters of the aforementioned acids, or mixtures thereof, do not fall under the definition of a conventional emulsifier according to the present invention.

According to the present invention, substantially emulsifier-free O/W emulsions are emulsions that contain less than 1.0 weight-% or less than 0.5 weight-%, preferably less than 0.3 weight-%, more preferred less than 0.1 weight-%, particularly preferred less than 0.05 weight-%, of conventional emulsifiers. According to the invention, emulsifier-free O/W emulsions are emulsions that do not contain any conventional emulsifiers.

According to the present invention, a solid emulsifier is a particulate substance that is wettable by both lipophilic and hydrophilic liquids. Solid emulsifiers may be inorganic or organic solids. Furthermore, the particles may be untreated or coated. The particle size is preferably between 1 nm and 200 nm, more preferred between 5 nm and 100 nm. If organic solid emulsifiers are used, such as for example crystalline fatty acids, crystalline fatty acid esters or crystalline fatty alcohols, the particle size may also amount to between 1 nm and 1000 nm.

According to the present invention, the at least one triterpenoid selected from the group consisting of betulin, betulinic acid, betulinic acid methyl ester, betulin aldehyde, betulonic acid, betulon aldehyde, lupeol, (C1-C6) alkyl esters of the aforementioned acids, or mixtures thereof, is at least partially present as particulate solid, if from about 10 weight-% to about 60 weight-% of the utilized amount of triterpenoid are present in the emulsion as particulate solid.

According to the present invention, the at least one triterpenoid selected from the group consisting of betulin, betulinic acid, betulinic acid methyl ester, betulin aldehyde, betulonic acid, betulon aldehyde, lupeol, (C1-C6), alkyl esters of the aforementioned acids, or mixtures thereof, is substantially present as particulate solid, if from about 60 weight-% to about 100 weight-% of the utilized amount of triterpenoid are present in the emulsion as particulate solid. According to the present invention, a lamellar arranged membrane is arranged such, that it possesses a layered structure such that the respective upper layer of the substance is oriented to a lower layer of the substance. The arrangement of the individual substance layers to each other occurs independently of the used solvent in a manner, that e.g. the hydrophilic moieties of the substance are directed outwards and the hydrophobic moieties are directed inwards to each other, or vice versa.

In case that two layers of the substance are oriented in the above-described manner, the resulting structure is designated as a single membrane, while in case of a superimposed arrangement of two further layers, this lamellar structure is designated as a double membrane. According to the present principle, still further layers may be associated to the already existing (double) membrane resulting in a multiple membrane structure. According to the present invention the membrane may be present as a single membrane, as a double membrane or also as a multiple membrane.

According to the invention, the term free acid or free acid functional group means compounds with an acid function, and an acid function, respectively, which are not neutralized to an extent of at least 98%, preferably at least 99%, particularly preferred of 100%.

According to the invention, the term completely neutralized acid or completely neutralized acid functional group means compounds with an acid function, and an acid function, respectively, which are neutralized and present in the form of their salts to an extent of at least 98%, preferably at least 99%, particularly preferred of 100%.

According to the invention, the term partially neutralized acid or partially neutralized acid functional group means compounds with an acid function, and an acid function, respectively, which are neutralized and present in the form of their salts to an extent of at least 2%, preferably at least 1%, and of at most 98%, preferably of at most 99%, while the non-neutralized fraction is present as a free acid.

"Stabilization of a foam formulation" may mean in the context of the present invention, that the formation of a foam is enabled which is applicable to the skin, and/or that the structure of the foam formed from the foam formulation can be maintained for a longer period of time, before the foam collapses, and/or that the foam formed from the foam formulation exhibits an increased strength. According to a further aspect, stabilization of a foam formulation may mean, that the structure of the foam can be maintained for a period of at least 30 seconds, preferably at least 1 minute, particularly preferred at least 2 minutes, before the foam collapses.

According to the invention, the expression "cyclic and linear N-vinyl carboxylic acid amides having a carbon chain of 2 to 9 carbon atoms" relates (i) in the case of cyclic amides to the number of carbon atoms in the cycle (e.g. in the case of N-vinylpyrrolidone the number of carbon atoms is 4), and (ii) in the case of linear amides to the chain length of the carboxylic acid moiety (e.g. in the case of N-vinyl acetamide, the respective number is 2).

### 2. Composition of the emulsion

The claimed O/W emulsion according to the present invention, in particular the emulsion underlying the foam formulations according to the present invention, which comprises an oil phase and an aqueous phase, comprises at least one triterpenoid selected from the group consisting of betulin, betulinic acid, betulinic acid methyl ester, betulin aldehyde, betulonic acid, betulon aldehyde, lupeol, (C1-C6)) alkyl esters of the aforementioned acids, or mixtures thereof. The emulsion is an oil-in-water emulsion. In a preferred embodiment the claimed emulsion comprises in addition to the at least one triterpenoid, an oil phase which comprises at least one membrane-forming substance forming a lamellar arranged membrane in the foam formulation.

In a further embodiment the claimed emulsion comprises in addition to the at least one triterpenoid at least one surface active, ionic polymer with a molecular weight of more than 5000 g/mol, wherein the ionic polymer is a copolymer comprising as monomer units an ionic monomer (M1), and at least one further monomer (also designated in the following as "emulsifier-copolymer").

In yet a further embodiment the claimed emulsion comprises in addition to the at least one triterpenoid at least one solid emulsifier. In further preferred embodiments the emulsion comprises in addition to the at least one triterpenoid combinations of the just mentioned further ingredients. In these embodiments the emulsion comprises in addition to the at least one triterpenoid:
aa) an oil phase which comprises at least one membrane-forming substance forming a lamellar arranged membrane in the foam formulation, and at least one emulsifier-copolymer; or
bb) an oil phase which comprises at least one membrane-forming substance forming a lamellar arranged membrane in the foam formulation, and at least one solid emulsifier; or
cc) at least one emulsifier-copolymer and at least one solid emulsifier; or
dd) an oil phase which comprises at least one membrane-forming substance forming a lamellar arranged membrane in the foam formulation, and at least one emulsifier-copolymer and at least one solid emulsifier.

The above embodiments can be advantageously combined with all of the further embodiments of the invention described in the following sections a) to k).

### a) Triterpenoid

In a preferred embodiment the at least one triterpenoid possesses a solubility in water of less than 0.5 % (w/w), more preferred less than 0.1 % (w/w), particularly preferred less than 0.05 % (w/w), more particularly preferred less than 0.01 % (w/w). The indicated values correspond to the solubility determined at room temperature.

In a further preferred embodiment the at least one triterpenoid possesses a solubility in water of less than 1 µg/ml, more preferred less than 0.75 µg/ml, particularly preferred less than 0.5 µg/ml, more particularly preferred less than 0.2 µg/ml. The indicated values correspond to the solubility determined at room temperature.

The skilled person is aware of methods for determining the water solubility of a substance. In particular the method for determining the water solubility of triterpenoids described in Jäger S. et al., Planta Med 2007; 73:157-62 and in Laszczyk, M., Triterpentrockenextrakt aus Birkenkork (Betula alba cortex), Untersuchungen zur chemischen Zusammensetzung, Galenik, Penetration und pharmakologisch-biologischen Wirkung, PhD thesis, Albert-Ludwigs-University, Freiburg, 2007, chapter 3.8, can be used.

In a preferred embodiment the at least one triterpenoid possesses a solubility in vegetable oil, preferably in refined sunflower oil, of less than 50 mg/ml, more preferred less than 20 mg/ml, particularly preferred less than 10 mg/ml, more particularly preferred less than 8 mg/ml, less than 5 mg/ml or even less than 3 mg/ml. The indicated values correspond to the solubility determined at room temperature.

The skilled person is also aware of methods for determining the solubility of a substance in oils. In particular the method for determining the solubility of triterpenoids in vegetable oil described in Laszczyk, M., Triterpentrockenextrakt aus Birkenkork (Betula alba cortex), Untersuchungen zur chemischen Zusammensetzung, Galenik, Penetration und pharmakologisch-biologischen Wirkung, PhD thesis, Albert-Ludwigs-University, Freiburg, 2007, chapter 3.7, can be used.

Preferably the at least one triterpenoid is at least partially or substantially present in the emulsion as a particulate solid. The skilled person knows how to achieve this, e.g. by the selection of the at least one triterpenoid, as well as the amount thereof in the emulsion.

In a further preferred embodiment the at least one triterpenoid used in the emulsion has an average particle size of less than 50 µm, preferably less than 10 µm, wherein the at least one triterpenoid preferably has a homogeneous particle size distribution. For example the particle size of the at least one triterpenoid may be in the range of from about 0.1µm to about 100 µm, preferably from about 0.2 µm to about 60 µm. Methods for obtaining triterpenoids with small average particle size are described e.g. in EP 1 758 555 B1 and in WO 01/72315 A1.

Preferably the emulsion comprises at least one triterpenoid selected from the group consisting of tetracyclic and pentacyclic triterpenoids or mixtures thereof. Particularly preferred the emulsion comprises at least one pentacyclic triterpenoid.

In a preferred embodiment the emulsion comprises at least one triterpenoid selected from the group of pentacyclic triterpenoids based on a lupane structure, an ursane structure, an oleanane structure, a friedelane structure, or mixtures thereof. In this respect the just mentioned compounds are particularly preferred the only triterpenoids in the emulsion.
Preferably the emulsion comprises at least one triterpenoid selected from the group consisting of betulin, betulinic acid, betulinic acid methyl ester, betulin aldehyde, betulonic acid, betulon aldehyde, lupeol, (C₁-C₆) alkyl esters of the aforementioned acids, or mixtures thereof. In this respect the just mentioned compounds are particularly preferred the only triterpenoids in the claimed emulsion. Particularly preferred the emulsion comprises at least one triterpenoid selected from the group consisting of betulin, betulinic acid, lupeol, (C₁-C₆) alkyl esters of the aforementioned acids, derivatives of the aforementioned compounds, or mixtures thereof. In this respect the just mentioned compounds are preferably the only triterpenoids in the claimed emulsion.

More particularly preferred the claimed emulsion comprises betulin and/or derivatives of betulin, preferably betulin.

The claimed emulsion preferably comprises at least 0.05 weight-% of the at least one triterpenoid, more preferred at least 0.08 weight-% of the at least one triterpenoid, particularly preferred at least 0.1 weight-% of the at least one triterpenoid, and more particularly preferred at least 0.2 weight-% of the at least one triterpenoid. For example, the emulsion may comprise from about 0.05 weight-% to about 10 weight-% of the at least one triterpenoid, preferably from about 0.08 weight-% to about 8 weight-% of the at least one triterpenoid, more preferably from about 0.1 weight-% to about 5 weight-% of at least one triterpenoid and particularly preferred from about 0.2 weight-% to about 3 weight-% of the at least one triterpenoid. The above values are each based on the total weight of the emulsion without propellant.

In a preferred embodiment the emulsion comprises at least 0.05 weight-% of betulin, more preferably at least 0.08 weight-% of betulin, particularly preferred at least 0.1 weight-% of betulin, and more particularly preferred at least 0.2 weight-% of betulin. For example, the emulsion may comprise from about 0.05 weight-% to about 10 weight-% of betulin, preferably from about 0.08 weight-% to about 8 weight-% of betulin, more preferably from about 0.1 weight-% to about 5 weight-% of betulin and particularly preferred from about 0.2 weight-% to about 3 weight-% of betulin. The above values are each based on the total weight of the emulsion without propellant.

The at least one triterpenoid may for example be present in form of at least one triterpenoid-containing plant extract.

Suitable plant extracts comprise birch cork extracts, extracts from the bark of Betula alba, extracts from the bark of Betula pendula, extracts from the bark of Betula platyphylla, extracts from the resin of the olibanum (incense) tree Boswellia, extracts from the resin of the myrrh tree Commiphora, extracts from the bark of the sycamore tree, mistletoe extracts, rosemary extracts, extracts from the roots and/or the leaves of the white ash Fraxinus americana, and extracts from the leaves and/or the bark of the mountain ash Sorbus americana.

If the at least one triterpenoid is present in form of a plant extract, the latter preferably comprises at least 40 weight-% of betulin, more preferably at least 50 weight-%, at least 60 weight-% or at least 70 weight-% of betulin (each based on the total weight of the plant extract). A particularly suitable triterpenoid-containing plant extract is for example the extract from the outer bark of Betula alba, Betula platyphylla or Betula pendula.

A suitable extract from the bark of Betula alba is for example available from the company Rita Corporation, Crystal Lake, USA, under the designation "Ritalab White Birch Bark Extract P" (CAS-no. 84012-15-7) in form of a white to off-white powder with a melting range of from 238 °C to 256 °C. This extract contains approximately 70 weight-% of betulin, as well as 4-10 weight-% of betulinic acid.

A further suitable triterpenoid-containing plant extract is available from the company Aromtech, Kiviranta, Finland, under the designation "Melabel Arctic Birch Bark Extract" (CAS-no. 85940-29-0). The latter is obtained by ethanolic extraction of the outer bark of Betula alba and Betula pendula and is present in form of a light beige-coloured powder. The powder contains at least 45 weight-% of betulin and at least 3 weight-% of lupeol.

In one embodiment, the emulsion underlying the foam formulations according to the present invention does not contain a combination of at least one pentacyclic triterpenoid and at least one cyclodextrin.

In alternative embodiments, the emulsion underlying the foam formulations according to the present invention does not contain the pentacyclic triterpenoid sericoside, or does not contain a combination of sericoside and at least one cyclodextrin.

Moreover, a cosmetic care emulsion, which is foamable by means of a manual pump dispenser, containing, in addition to at least one oil phase and at least one aqueous phase, an emulsifier combination of at least one W/O-emulsifier having a HLB value of 4 to 6, and at least one O/W-emulsifier compound having a HLB value in the range of 10 to 14, wherein both the W/O and the O/W-emulsifier compound contain a poly(12-hydroxy stearic acid) poly glycerol ester, and wherein the emulsion further comprises oleanolic acid and/or oleanol, is preferably excluded from the foam formulations according to the present invention.

### b) Oil phase

Suitable components that may form the oil phase may be selected from polar and non-polar lipids or mixtures thereof.

The oil phase of the inventive formulations is advantageously selected from the group of phospholipids, such as lecithins, (mono-, di-, tri-) glycerides (in particular triglycerides, such as e.g. fatty acid triglycerides), sphingolipids, from the group of propylene glycol fatty acid esters or butylene glycol fatty acid esters, from the group of natural waxes of animal or vegetable origin, from the group of ester oils, from the group of dialkyl ethers and dialkyl carbonates, from the group of branched and unbranched hydrocarbons and waxes as well as from the group of cyclic and linear silicon oils.

In one embodiment the oil phase comprises at least one fatty acid alkyl ester such as for example oleic acid decyl ester (decyl oleate) or cetearyl isononanoate, and/or at least one fatty alcohol such as for example 2-octyldodecanol. Furthermore, the oil phase may contain saturated aliphatic hydrocarbons such as paraffin.

Decyl oleate is available for example from the company Cognis under the designation Cetiol V. Cetearyl isononanoate is available for example from the company Cognis under the designation Cetiol SN. 2-Octyldodecanol is available for example from the company Cognis under the designation Eutanol G.

In a preferred embodiment, the oil phase comprises at least one triglyceride. Preferably, the at least one triglyceride comprises caprylic acid/capric acid triglyceride, obtainable under the designation Miglyol 812 of the company Sasol, and admixtures thereof with further oil and wax components. Furthermore, particularly preferred are triglycerides, in particular caprylic acid/capric acid triglyceride obtainable under the designation Miglyol 812 of the company Sasol/Myritol 312 of the company Cognis.

Moreover the oil phase may preferably contain further ingredients, such as e.g. fatty acids, in particular stearic acid. The oil phase may preferably contain vegetable oils, such as for example almond oil, avocado oil, sunflower oil, or olive oil.

The emulsions according to the invention preferably contain from 5 to 50 weight-% oil phase, particularly preferred 10 to 35 weight-% and more preferred 15 to 35 weight-% oil phase. These values are each based on the total weight of the emulsion without propellant.

### c) Membrane-forming substances in the oil phase

In preferred embodiments the oil phase of the emulsion comprises at least one membrane-forming substance forming a lamellar arranged membrane in the foam formulation. The at least one membrane-forming substance may be a single compound or a mixture of substances, preferably a mixture of substances.

Preferably the membrane-forming substances of the invention have a HLB-value of more than 8, more preferably of from 9 to 11, and most preferably of from 9.5 to 10.5.

The membrane-forming substances and mixtures of substances according to the present invention are typically water insoluble, while conventional emulsifiers, in particular surfactants having a comparable HLB-value of about 10, are generally water soluble. Furthermore, the water insoluble, membrane-forming substances according to the present invention are not capable of spontaneously emulsifying oils, while conventional emulsifiers, in particular those having a high HLB-value, are capable of spontaneously emulsifying oils. Conventional emulsifiers having a low HLB-value are not capable of forming lamellar structures or liposomes on their own, in contrast to membrane-forming substances according to the present invention, e.g. phospholipids.

Membrane-forming substances according to the present invention normally do not form micelles or hexagonal liquid crystalline phases on their own. Above the phase transition temperature, they spontaneously form exclusively large multilamellar vesicles in water. Below the phase transition temperature, they can be dispersed in water under high energy input and form lamellar structures. The mentioned phase transition temperature indicates the temperature at which a gel-like phase converts into a liquid crystalline phase. Below the phase transition temperature, a gel phase is present, above the phase transition temperature, a liquid crystalline phase is present. The phase transition temperatures vary depending on the composition (saturated/unsaturated; short/long) and for example in the case of phospholipids, they typically amount to between 10 °C and 70 °C. For a given system, the phase transition temperature can be easily determined by means of DSC.

According to the present invention, a membrane-forming substance forming a lamellar arranged membrane is a substance that preferably has both a hydrophilic and a hydrophobic molecular moiety. The capacity of a membrane-forming substance to form lamellar structures instead of micelles depends, however, in particular on the optimal area aₒ (interface carbon/water), the volume V and the critical chain length l_{c} (Israelachvili, Jacob N.: "Intermolecular and Surface Forces: With Applications to Colloidal and Biological Systems". 2nd Edition Academic Press, London, UK, 1992). Furthermore, it may perhaps be necessary to select specific manufacturing conditions, so that a system forms lamellar structures. Suitable conditions are described further below.

Examples for membrane-forming substances are e.g. phospholipids, such as lecithins, sphingolipids, ceramides, cholesterol, fatty alcohols, fatty acids as well as mono-and/or diesters thereof, and sterols. Triglycerides (not hydrophilic and lipophilic), squalene (not hydrophilic and lipophilic), squalane (not hydrophilic and lipophilic), may also be contained in mixtures of substances comprising membrane-forming substances.

Preferably the at least one membrane-forming substance comprises phospholipids, sphingolipids, ceramides, cholesterol, fatty alcohols, fatty acids as well as mono-and/or diesters thereof, and sterols.

Such substances or corresponding mixtures of substances can be dispersed under suitable conditions with an aqueous phase resulting in the formation of lamellar membranes. This can be achieved, for example, by dispersing under high energy input (e.g. high pressure homogenization, ultrasound), as described further below. It is also possible to use certain, commercially available prefabricated concentrates which form a lamellar phase.

In a preferred foam formulation of the invention, the membrane-forming substance comprises a lipid, more preferably a triglyceride and/or a phospholipid. In a particularly preferred foam formulation of the invention, the triglyceride is caprylic acid/capric acid triglyceride, and/or the phospholipid is lecithin, preferably hydrogenated lecithin.

In a preferred foam formulation of the present invention, the at least one membrane-forming substance comprises lecithin, preferably hydrogenated lecithin.

Preferably the emulsion further comprises further ingredients, such as stabilizers such as e.g. alcohols or glycols. Preferred are glycols, in particular propylene glycol, caprylyl glycol or mixtures thereof

In further preferred embodiments the emulsion comprises further ingredients such as e.g. butyrospermum parkii (shea butter), squalane, glycerides, ceramides, preferably ceramide 3, or mixtures thereof

According to the present invention, the oil phase which comprises a substance suitable for the formation of lamellar arranged membranes, or a mixture of such substances, is dispersed with an aqeuous phase under conditions that result in the formation of a lamellar phase. If necessary, this is done, for example, by dispersing under high energy input, such as e.g. by ultrasound or by means of high pressure homogenization, wherein pressures of about 50,000 to about 250,000 kilopascal (about 500 to about 2500 bar), preferably about 100,000 to about 150,000 kilopascal (about 1000 to about 1500 bar) are used. In other cases, in particular when using non-hydrogenated lecithin having a low phase transition temperature as the membrane-forming substance, simple dispersing is often sufficient, without the additional need of a high energy input.

The presence of lamellar structures in the dispersion can be easily determined by the skilled person using methods known in the art. Suitable measuring methods are, for example, described in Claus-Dieter Herzfeld et al. (eds.), Grundlagen der Arzneiformlehre, Galenik 2, Springer publishing, 1999. In this respect, the method of polarization microscopy is particularly worth mentioning. In this method, two polarization films are placed above and below the object to be analyzed in the so-called cross position, wherein the oscillation planes of the generated polarized light are perpendicular to each other. The oscillation plane of the irradiated light is changed by the sample so that a fraction of the light can pass through the second polarization film. The presence of lamellar phases can typically be recognized here by so-called Maltese crosses.

In a particularly preferred embodiment, the membrane-forming substance comprises a phospholipid, such as lecithin or hydrogenated lecithin, and additionally a further lipid. More preferably, the phospholipid is a mixture of lecithin and hydrogenated lecithin. In a particularly preferred embodiment, the weight ratio of lecithin to hydrogenated lecithin is about 10:1 to about 1:10, more preferably about 5:1 to about 1:5 and still more preferably the ratio of lecithin to hydrogenated lecithin is about 1:1. The lipid present in addition to the phospholipid comprises in a preferred embodiment a liquid wax ester, such as e.g. isopropyl myristate, isopropyl palmitate, isopropyl stearate, or the like. Moreover, further lipids, such as e.g. peanut oil or medium chain triglycerides (preferably C₈-C₁₂ triglycerides), may optionally be present in addition to the wax ester. In this embodiment, the weight ratio of total phospholipid (e.g. such as lecithin + hydrogenated lecithin) to total lipid (e.g. wax ester + optional triglycerides) preferably amounts to about 1:5 to about 1:1, preferably about 1:2.

The mixture of phospholipid and lipid is, for example, dispersed as a melt with water under high energy input. The high energy input can be effected by means of ultrasound or high pressure homogenization, wherein pressures of 50,000 to 250,000 kilopascal (500 to 2500 bar), preferably 100,000 to 150,000 kilopascal (1000 to 1500 bar), are employed. In the aqueous phase, further additives may optionally be present as described in the present specification, such as glycerol or thickening agents (e.g. xanthan gum and/or hydroxypropylmethyl cellulose (hypromellose).

Commercially available base creams using the above-described "skin-like" ingredients, are also known in the art as DMS^{®} base creams. The DMS^{®} base creams are emulsions containing membrane-forming substances, and are typically obtained by high pressure homogenization. As cream basis, DMS® base creams can be stirred, e.g. by pharmacists or by the further processing cosmetics industry, into existing formulations, and therein form the above described lamellar structures (see e.g. Österreichische Apothekerzeitung, 56 (14), 679 (2002)).

As major components DMS-base creams contain for example water, hydrogenated lecithin, fatty substances such as triglycerides and squalane, phytosterols, e.g. from shea butter, and moisturizing agents such as e.g. glycerol (see e.g. Osterreichische Apothekerzeitung, 56 (14), 679 (2002)).

In particular, the DMS^{®} base compositions can have the following ingredients: caprylic acid/capric acid triglyceride, shea butter, squalane, ceramide 3, hydrogenated lecithin, palm glycerides, persea gratissima, palm oil (elaesis guineensis). As stabilizers, e.g. alcohols or glycols such as e.g. pentylene glyclol, caprylyl glycol or mixtures thereof, may be used in the DMS^{®} compositions. Preferably, further (non body-identical) conventional additives such as fragrances, colorants, comedogenic lipids (e.g. mineral oils) and physiologic emulsifiers are omitted in the DMS concentrates and in the inventive formulations, since these components are potentially sensitizing and may cause skin irritations.

The composition of various, commercially available DMS^{®} base creams is described in WO 2008/155389 on pages 22 to 23, the disclosure of which is hereby incorporated by reference.

A preferred DMS-base comprises the following composition: water, hydrogenated lecithin, caprylic acid/capric acid triglyceride, pentylene glycol, butyrospermum parkii butter, glycerol, squalane, ceramide 3. It is available from the company KUHS under the designation Probiol.

In preferred embodiments, wherein the at least one membrane-forming substance is used for example in form of a DMS-base cream, the latter comprises the following ingredients:
- caprylic acid/capric acid triglyceride, shea butter, squalane, ceramide 3, hydrogenated lecithin as well as pentylene glycol; or
- caprylic acid/capric acid triglyceride, shea butter, squalane, ceramide 3, hydrogenated lecithin as well as alcohol, or
- caprylic acid/capric acid triglyceride, shea butter, squalane, ceramide 3, hydrogenated lecithin, persea gratissima as well as caprylyl glycol, or
- caprylic acid/capric acid triglyceride, shea butter, squalane, ceramide 3, hydrogenated lecithin, palm glyceride, elaesis guineensis as well as pentylene glycol, or
- caprylic acid/capric acid triglyceride, butyrospermum parkii, squalane, ceramide 3, hydrogenated lecithin, as well as pentylene glycol, or
- hydrogenated lecithin, caprylic acid/capric acid triglyceride, pentylene glycol, butyrospermum parkii butter, glycerol, squalane, ceramide 3.

Foam formulations according to the present invention, which comprise at least one membrane-forming substance, allow for an improved skin care action of the formulation, due to the resulting lamellar membrane structure and the resulting structural similarity to the composition of the intercellular lamellar lipid structure of epidermal lipids, especially of the *stratum corneum*. Due to the analogous structure to the lamellar structure of the skin, an easier integration of the membrane into the skin is achieved. The integration also leads to an improvement, especially of the stabilization and the reconstruction of the skin barrier. An intact skin barrier protects the skin from excessive moisture loss. An improvement of the skin barrier can also effect an improved skin smoothing and decrease the "wash-out" effect, whereby advantageously an improved long-term effect is achieved in comparison to conventional foam formulations.

### d) Presence of conventional emulsifiers

In one embodiment the emulsion is free from the following compounds:
- carboxylates, such as e.g. sodium stearate, aluminium stearate;
- sulfates, such as e.g. Na-dodecyl sulfate, Na-cetyl stearyl sulfate, Nalaurylether sulfate;
- sulfonates, such as e.g. Na-dioctyl sulfosuccinate;
- quaternary ammonium compounds, such as e.g. cetyl trimethyl ammonium bromide, benzalkonium bromide;
- pyridinium compounds, such as e.g. cetyl pyridinium chloride;
- betaines, such as e.g. betaine monohydrate;
- macrogol fatty acid esters, such as e.g. macrogol-30-stearate;
- glycerol fatty acid esters, such as e.g. glycerol monostearate, glycerol monooleate, glycerol monoisostearate, partial glycerides of medium chain length;
- polyoxyethylene sorbitan fatty acid esters, such as e.g. Tween^{®}, polyoxyethylene-(20)-sorbitan monostearate;
- sorbitan fatty acid esters, such as e.g. sorbitan laurate, sorbitan monooleate, sorbitan monopalmitate, sorbitan monostearate, sorbitan tristearate, sorbitan sesquioleate;
- sucrose fatty acid esters, such as e.g. sucrose monostearate, sucrose distearate, sucrose cocoate;
- macrogol fatty alcohol ethers, such as e.g. cetomacrogol 1000, macrogol cetostearyl ether, macrogol oleyl ether, Lauromacrogol 400;
- sterol alcohols, such as e.g. cholesterol, lanolin, acetylated lanolin, hydrogenated lanolin, lanolin alcohols;
- macrogol glycerol fatty acid esters, such as e.g. macrogol-1000-glycerol-monooleate, macrogol-1000-glycerol-monostearate, macrogol-1500-glycerol-triricinoleate, macrogol-300-glycerol-tris(hydroxy stearate), macrogol-5-glycerol-stearate, macrogol glycerol hydroxystearate;
- Polyglyercol fatty acid esters, such as e.g. triglycerol diisostearate.

In a further embodiment the emulsion is free from water soluble, conventional emulsifiers having a HLB value of about 10.

In a preferred embodiment the foam formulation comprises a substantially emulsifier-free emulsion. In a particularly preferred embodiment the foam formulation comprises an emulsifier-free emulsion.

### e) Emulsifter-copolymer

In further preferred embodiments the emulsion further comprises at least one surface active, ionic polymer with a molecular weight of more than 5000 g/mol, wherein the ionic polymer is a copolymer, comprising as monomer units an ionic monomer (M1) and at least one further monomer. In the following, such polymers are also referred to as "emulsifier-copolymer".

The at least one emulsifier-copolymer is preferably water soluble or water swellable, particularly preferred water swellable. In the context of the present invention, "water swellable" means that upon contact with water, the polymer hydrates accompanied by a volume increase.

Preferably, the emulsion contains the at least one emulsifier-copolymer in an amount of from about 0.01 to about 5 weight-%, preferably from about 0.01 to about 1 weight-%, more preferably from about 0.01 to about 0.5 weight-%, and particularly preferred from about 0.01 to about 0.1 weight-% (each based on the total weight of the emulsion without propellant).

An emulsifier-copolymer which contains one further monomer is a bipolymer, in the case of two further monomers it is a terpolymer, etc. According to the present invention, bipolymers, terpolymers, quaterpolymers, etc. are all comprised by the term copolymer.

The at least one further monomer is different from the ionic monomer (M1). Preferably, the at least one further monomer has a different polarity than the ionic monomer (M1).

The at least one further monomer is preferably selected from the group consisting of ionic monomers, non-ionic monomers and mixtures thereof. Particularly preferred, the at least one further monomer comprises at least one non-ionic monomer.

### aa) Ironic monomer (M1):

The ionic monomer (M1) is preferably anionic, cationic or zwitterionic, particularly preferred anionic.

Preferably, the ionic monomer (M1) contains free, partially neutralized or completely neutralized acid functional groups. A monomer containing free acid functional groups is to be understood as ionic monomer because the acid functional groups may be at least partially neutralized either during the manufacture of the copolymer or during the manufacture of the oil-in-water emulsion.

The acid functional groups are preferably selected from the group consisting of sulfonic acid groups, carboxylic acid groups, phosphoric acid groups, phosphonic acid groups and mixtures thereof.

In a preferred embodiment, the ionic monomer (M1) is selected from the group consisting of acrylic acids, methacrylic acids, crotonic acids, maleic acids, fumaric acids, styrene sulfonic acids, vinyl sulfonic acids, vinyl phosphonic acids, allyl sulfonic acids, methallyl sulfonic acids, acrylamido alkylsulfonic acids, which may each be present as free acid, partially or completely neutralized in the form of their salts, preferably the alkali metal salts, alkaline-earth metal salts, ammonium salts or alkanol ammonium salts; or as anhydride, and mixtures thereof. In a particular preferred embodiment, the ionic monomer (M1) is selected from the group consisting of acrylic acids, methacrylic acids, and acrylamido alkylsulfonic acids.

In this respect, the ionic monomer (M1) is preferably an acrylamido alkylsulfonic acid, such as for example 2-acrylamido-2-methylpropane sulfonic acid. Particularly preferred, the acrylamido alkylsulfonic acid is present partially or completely neutralized as alkali metal salt, alkaline-earth metal salt, ammonium salt or alkanol ammonium salt, particularly preferred as sodium or ammonium salt, most preferred as ammonium salt.

Particularly preferred, the ionic monomer (M1) is an acrylamido alkylsulfonic acid having the general formula (1), 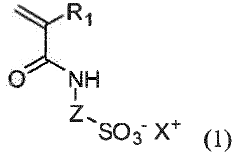 wherein R₁ is selected from the group consisting of hydrogen, methyl or ethyl, Z is a (C₁-C₈)-alkylene, that may be unsubstituted or substituted with one or more (C₁-C₄)-alkyl groups, and X⁺ is selected from the group consisting of H⁺, an alkali metal ion, an alkaline-earth metal ion, an ammonium ion, an alkanol ammonium ion, or mixtures thereof. Preferably, X⁺ is selected from the group consisting of H⁺, Na⁺, NH₄⁺, or mixtures thereof.

In a particularly preferred embodiment the ionic monomer (M1) is 2-acrylamido-2-methylpropane sulfonic acid (AMPS, 2-methyl-2-[(1-oxo-2-propenyl)amino]-1-propanesulfonic acid), having the chemical formula (2), 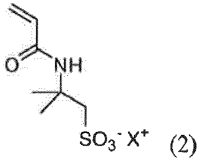 and may be present as free acid (X⁺ is H⁺), or partially or completely neutralized in the form of its salts (the acryloyldimethyltaurates, X⁻¹ is a cation except H⁺, e.g. an alkali metal ion such as Na⁺, an alkaline-earth metal ion such as (1/2) Ca²⁺, or an ammonium ion, such as NH₄⁺). Preferably, X⁺ is selected from the group consisting of H⁺, Na⁺, NH₄⁺, or mixtures thereof. Particularly preferred, the ionic monomer (M1) is sodium acryloyldimethyltaurate or ammonium acryloyldimethyltaurate (X⁺ is Na⁺ and NH₄⁺, respectively).

In alternative embodiments, the ionic monomer (M1) is an acrylic acid and/or a methacrylic acid.

### bb) Further monomer:

In one embodiment, the at least one further monomer comprises at least one non-ionic monomer, preferably selected from the group consisting of styrenes, chlorostyrenes, di-(C₁-C₃₀)-alkylamino styrenes, vinyl chlorides, isoprenes, vinyl alcohols, vinyl methyl ethers, (C₁-C₃₀)-carboxylic acid vinyl esters, preferably vinyl acetates and vinyl propionates; acrylic acid esters, methacrylic acid esters, maleic acid esters, fumaric acid esters, crotonic acid esters; in particular linear and branched (C₁-C₃₀)-alkyl esters of acrylic acid, methacrylic acid, maleic acid, fumaric acid and crotonic acid; linear and branched (C₁-C₃₀)-hydroxyalkyl esters of acrylic acid, methacrylic acid, maleic acid, fumaric acid and crotonic acid; ethoxylated (C₁-C₃₀)-alkyl esters of acrylic acid, methacrylic acid, maleic acid, fumaric acid and crotonic acid with from 1 to 40 ethylene oxide units; acrylamides, in particular N,N-di-(C₁-C₃₀)-alkyl acrylamides, methacrylamides, in particular N,N-di-(C₁-C₃₀)-alkyl methacrylamides, cyclic and linear N-vinyl carboxylic acid amides with a carbon chain of 2 to 9 carbon atoms, preferably N-vinylpyrrolidone; and mixtures thereof

In a preferred embodiment the at least one further monomer comprises a non-ionic monomer selected from the group consisting of linear and branched (C₁-C₆)-hydroxyalkyl esters of acrylic acid or methacrylic acid, preferably hydroxyethyl acrylate; ethoxylated (C₁-C₃₀)-alkyl esters of acrylic acid or methacrylic acid having from 1 to 40 ethylene oxide units; preferably beheneth-25-methacrylate; N,N-di-(C₁-C₆)-alkyl acrylamides, preferably N,N-dimethylacrylamides, cyclic and linear N-vinyl carboxylic acid amides having a carbon chain of 2 to 9 carbon atoms, preferably N-vinylpyrrolidone, and mixtures thereof.

The at least one further monomer may also comprise at least one ionic monomer, preferably selected from the group consisting of acrylic acids, methacrylic acids, crotonic acids, maleic acids, fumaric acids, styrene sulfonic acids, vinyl sulfonic acids, vinyl phosphonic acids, allyl sulfonic acids, methallyl sulfonic acids, acrylamido alkylsulfonic acids, which may each be present as free acid, partially or completely neutralized in the form of their salts, preferably the alkali metal salts, alkaline-earth metal salts or ammonium salts; or as anhydride, and mixtures thereof. In a preferred embodiment, the at least one further monomer comprises an acrylic acid, that is present partially or completely neutralized in the form of its alkaline metal salt, alkaline-earth metal salt or ammonium salt. Particularly preferred, the at least one further monomer comprises sodium acrylate.

### cc) Preferred emulsifier-copolymers

A particularly suitable emulsifier-copolymer is for example sodium acrylate/sodium acryloyldimethyltaurate copolymer, in particular in the form of the product available from the company Seppic under the designation 8885MP2 (Sepinov EG-P). Another particularly suitable emulsifier-copolymer is sodium acrylate/ acryloyldimethyltaurate/ dimethylacrylamide crosspolymer, in particular in the form of the product available from the company Seppic under the designation 8732MP (Sepinov P88). A further particularly suitable emulsifier-copolymer is hydroxyethyl acrylate/ sodium acryloyldimethyltaurate copolymer, particularly in the form of the product markteted by the company Seppic under the trade name Sepinov™ EMT 10.

A particularly suitable emulsifier-copolymer is acryloyldimethyltaurate/ vinylpyrrolidone copolymer, preferably ammonium acryloyldimethyltaurate/ vinylpyrrolidone copolymer, in particular in the form of the product marketed under the trade name Aristoflex® AVC.

In a particularly preferred embodiment the at least one emulsifier-copolymer comprises acryloyldimethyltaurate/ vinylpyrrolidone copolymer having the general formula (3) 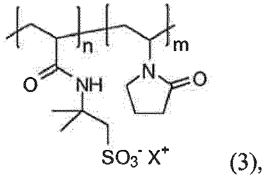 wherein X⁺ is Na⁺ or NH₄⁺, and n and m are integers that vary independently from each other between 1 to 10,000. In this respect, the polymer is preferably a statistical copolymer, a block copolymer or a graft copolymer, particularly preferred a statistical copolymer.

In alternative preferred embodiments, wherein the ionic monomer (M1) is an acrylic acid and/or a methacrylic acid, the at least one further monomer is preferably selected from the group consisting of cyclic and linear N-vinyl carboxylic acid amides having a carbon chain of 2 to 9 carbon atoms, linear and branched (C₁-C₃₀)-alkyl esters of acrylic acid, linear and branched (C₁-C₃₀)-alkyl esters of methacrylic acid, linear and branched (C₁-C₃₀)-hydroxyalkyl esters of acrylic acid, linear and branched (C₁-C₃₀)-hydroxyalkyl esters of methacrylic acid, and mixtures thereof. In particular, the at least one further monomer may be selected from the group consisting of cyclic and linear N-vinyl carboxylic acid amides having a carbon chain of 2 to 9 carbon atoms, linear and branched (C₁-C₆)-alkyl esters of acrylic acid, linear and branched (C₁-C₆)-alkyl esters of methacrylic acid, and mixtures thereof.

In a preferred embodiment, the ionic monomer (M1) is acrylic acid, and the at least one further monomer is a cyclic or linear N-vinyl carboxylic acid amide having a carbon chain of 2 to 9 carbon atoms. Such copolymers, their preparation and use in hairstyling products are described e.g. in WO 2006/044193 A2.

The cyclic or linear N-vinyl carboxylic acid amides mentioned above are preferably selected from the group consisting of N-vinyl pyrrolidone, N-vinyl caprolactame, N-vinyl acetamide, or N-vinyl-N-methylacetamide. Preferably the N-vinyl carboxylic acid amide having a carbon chain of 2 to 9 carbon atoms is N-vinyl pyrrolidone.

A particularly suitable emulsifier-copolymer in this regard is for example acrylic acid/ N-vinyl pyrrolidone copolymer, in particular in the form of the product UltraThix P-100 (INCI-name: acrylic acid/VP Crosspolymer). UltraThix P-100 which is a lightly crosslinked copolymer of vinyl pyrrolidone and acrylic acid is marketed by the company ISP.

The weight ratio of acrylic acid to N-vinyl pyrrolidone in the acrylic acid/N-vinyl pyrrolidone copolymer may preferably be in the range of 1:3 to 3:1, more preferably of 1:2 to 2:1, most preferably is equal to 1:1.

In a further preferred embodiment, the ionic monomer (M1) is methacrylic acid and the at least one further monomer is selected from one or more, linear or branched (C₁-C₆)-alkyl esters of acrylic acid or methacrylic acid, preferably from one or more linear or branched (C₁-C₆)-alkyl esters of acrylic acid, more preferably from one or more of methyl acrylate, ethyl acrylate, n-propyl acrylate, i-propyl acrylate, n-butyl acrylate, i-butyl acrylate, tert-butyl acrylate, and mixtures thereof.

A particularly suitable emulsifier-copolymer in this regard is for example a terpolymer of tert-butyl acrylate, ethyl acrylate and methacrylic acid, in particular in the form of the product Luvimer® 100 P (INCI-name: Acrylates copolymer). Polymers of the Luvimer®) series (such as Luvimer® 100 P, Luvimer® 36 D and Luvimer® 30 E) are marketed by the company BASF SE.

The applicant has furthermore surprisingly found that particularly stable and fine-pored foams can be obtained when the emulsions and/or foam formulations according to the present invention contain a combination of
a) a copolymer of acrylic acid and a cyclic or linear N-vinyl carboxylic acid amide having a carbon chain of 2 to 9 carbon atoms, such as acrylic acid/ N-vinyl pyrrolidone copolymer and
b) a copolymer of methacrylic acid and one or more linear or branched (C₁-C₆)-alkyl esters of acrylic acid, such as tert-butyl acrylate/ ethyl acrylate/ methacrylic acid terpolymer.

Hence in preferred embodiments of the present invention the at least one surface active, ionic polymer comprises acrylic acid/ N-vinyl pyrrolidone copolymer and/or tert-butyl acrylate/ ethyl acrylate/ methacrylic acid terpolymer.

According to a further aspect of the present invention the at least one surface active, ionic polymer is preferably selected from the group consisting of acryloyldimethyltaurate/ vinylpyrrolidone copolymer, sodium acrylate/ acryloyldimethyltaurate/ dimethylacrylamide crosspolymer, hydroxyethyl acrylate/ sodium acryloyldimethyltaurate copolymer, sodium acrylate/ sodium acryloyldimethyltaurate copolymer, acrylic acid/ N-vinyl pyrrolidone copolymer, tert-butyl acrylate/ ethyl acrylate/ methacrylic acid terpolymer, and mixtures thereof.

Preferably, the at least one emulsifier-copolymer is used in pre-neutralized form, wherein it is preferably present in powder form. Alternatively, the emulsifier-copolymers may at least be partially neutralized during the production of the emulsion, e.g. by adjusting the pH of an aqueous phase containing the emulsifier-copolymer.

The emulsifier-copolymer may be present for example as a statistical copolymer, as a block copolymer or as a graft copolymer, or mixtures thereof, wherein statistical copolymers are preferred.

In specific embodiments of the present invention, the emulsifier-copolymer is cross-linked, wherein the crossed-linked emulsifier-copolymer contains preferably from 0.001 to 10 weight-%, particularly preferred 0.01 to 10 weight-% of crosslinking agent.

As crosslinking agents may be used for example diallyloxyacetic acid or its salts, trimethylol propane triacrylate, trimethylol propane diallyl ether, ethylene glycol dimethacrylate, diethylene glycol diacrylate, tetraethylene glycol diacrylate, methylene bis(acrylamide), divinylbenzene, diallyl urea, triallylamine, 1,1,2,2-tetraallyloxyethane, acrylic acid allyl ester, methacrylic acid allyl ester, dipropyleneglycol diallyl ether, polyglycol diallyl ether, triethyleneglycol divinylether, or hydrochinone diallyl ether. Other suitable crosslinking agents comprise pentaerythritol triallylether, pentaerythritol triacrylate, or pentaerythritol tetraacrylate.

In a particularly preferred embodiment of the present invention, the at least one surface active, ionic polymer comprises a linear acrylic acid/N-vinyl pyrrolidone copolymer, which is crosslinked with 0,5 to 1,5 weight-%, preferably with about 1 weight-% of pentaerythritol triallylether.

In the context of the present invention, copolymers containing intramolecular cross-linkages are referred to as "cross-linked copolymers" or "cross-polymers".

### f) Solid emulsifier

In further preferred embodiments, the emulsion further comprises at least one solid emulsifier.

Preferably the emulsion comprises the at least one solid emulsifier in an amount of more than 0.5 weight-%, particularly preferred more than 1 weight-%. In particular, the emulsion may contain from 0.5 to 7 weight-%, preferably from 0.5 to 5 weight-%, particularly preferred from 0.5 to 3 weight-% of the at least one solid emulsifier. The weight percentages are each based on the total weight of the emulsion without propellant.

Suitable solid emulsifiers are particulate inorganic or organic solids that are wettable by both lipophilic and hydrophilic liquids. Suitable representatives are e.g. titanium dioxide, in particular coated titanium dioxide (e.g. obtainable from Merck KGaA under the designation Eusolex® T-2000), zinc oxide (e.g. obtainable from BASF SE under the designation Z-Cote Max), silicon dioxide, in particular highly disperse silicon dioxide, Fe₂O₃, veegum, bentonite and ethyl cellulose. Furthermore, aluminium oxide, calcium carbonate, coal, magnesium oxide, magnesium trisilicate, crystalline fatty acids, crystalline fatty acid esters, crystalline fatty alcohols, polymer lattices, e.g. polystyrenes or polymethacrylates and polymer-pseudo lattices may be used. Mixtures of the aforementioned solid emulsifiers may also be used. Preferably, the at least one solid emulsifier is selected from the group consisting of crystalline fatty acids, crystalline fatty acid alkyl esters, crystalline fatty alcohols or mixtures thereof.

Preferably, the at least one solid emulsifier comprises a crystalline fatty acid, preferably with a chain length of 10 to 40 carbon atoms. The crystalline fatty acid is in particular a saturated fatty acid, preferably selected from the group consisting of myristic acid, palmitic acid, margaric acid, stearic acid and arachidic acid or mixtures thereof.

In a particularly preferred embodiment, the at least one solid emulsifier comprises stearic acid. Stearic acid is available for example from the company Cognis under the name Cutina FS 45.

Furthermore, the at least one solid emulsifier may comprise a crystalline fatty alcohol, preferably with a chain length of 10 to 40 carbon atoms. The crystalline fatty alcohol is in particular a saturated fatty alcohol, preferably selected from the group consisting of myristyl alcohol, cetyl alcohol, heptadecanol, stearyl alcohol, cetylstearyl alcohol, eicosanol or mixtures thereof.

### 2) Aqueous phase

The aqueous phase may contain cosmetic adjuvants, e.g. lower alcohols (e.g. ethanol, isopropanol), lower diols or polyols as well as ethers thereof (e.g. propylene glycol, glycerol, butylene glycol, hexylene glycol and ethylene glycol), foam stabilizers and thickeners.

Suitable thickeners are polymeric thickeners that are partially water soluble or at least water dispersible and that form in aqueous systems gels or viscous solutions. They increase the viscosity of the water either by binding water molecules (hydration) or, by incorporating and encapsulating the water into their interwoven macromolecules, wherein the mobility of the water is decreased. Suitable polymers are:
- Modified natural substances, such as cellulose ethers (e.g. hydroxypropyl cellulose ether, hydroxyethyl cellulose and hydroxypropyl methyl cellulose ether);
- Natural compounds, such as e.g. xanthan, agar-agar, carrageen, polyoses, starch, dextrines, gelatine, casein;
- Synthetic compounds, such as e.g. vinyl polymers, polyethers, polyimines, polyamides and derivates of polyacrylic acid; and
- Inorganic compounds, such as e.g. polysilicic acid and clay minerals.

Preferably, the emulsion contains at least one thickener selected from the group consisting of hydroxypropyl methyl cellulose, xanthan gum, sodium polyacrylate and mixtures thereof. Particularly preferred the emulsion contains xanthan gum and/or sodium polyacrylate as thickener.

Xanthan gum is available for example from the company Kelco under the designation Keltrol® CG. Sodium polyacrylate is available for example from the company Cognis under the designation Cosmedia SP.

The emulsion preferably contains from 0.01 to 1.5 weight-% of thickener (based on the dry weight of the thickener and the total weight of the emulsion without propellant). Particularly preferred are 0.02 to 1.0 weight-% of thickener. More particularly preferred are 0.02 to 0.5 weight-% of thickener.

In a preferred embodiment the emulsion comprises from 0.01 to 1.0 weight-%, preferably from 0.01 to 0.5 weight-% of xanthan gum, and/or from 0.01 to 1.0 weight-%, preferably from 0.01 to 0.5 weight-% of sodium polyacrylate (each based on the dry weight of the thickener and the total weight of the emulsion without propellant).

### h) Active agents

The emulsion may also comprise one or more active agents. The optionally contained active agent may be selected from all active agents that can be applied to the surface of the skin, and mixtures thereof. The active agent may act cosmetically or pharmaceutically. Accordingly, cosmetic or dermatological foam formulations (to be employed as medical product or medicament) are obtained. Furthermore, the formulation may be used for protecting the skin against environmental influences. The active agent can be of natural or synthetic origin. The group of active agents may also overlap with the other groups of ingredients, such as e.g. the oil component, or the optionally contained thickening agents or solid emulsifiers. For example, some oil components may also act as active agents, such as e.g. oils having polyunsaturated fatty acids, or solid emulsifiers, such as e.g. particulate titanium dioxide may serve as UV-filter. Depending on their properties, the substances can be assigned to several groups.

In particular the at least one triterpenoid contained in the emulsion can simultaneously act as active agent, e.g. when the at least one triterpenoid comprises betulin or betulinic acid.

Active agents of the inventive formulations are advantageously selected from the group of substances having moisturizing and barrier-strengthening properties, such as e.g. Hydroviton, an emulation of NMF, pyrrolidone carboxylic acid and salts thereof, lactic acid and salts thereof, glycerol, sorbitol, propylene glycol and urea, substances from the group of proteins and protein hydrolysates, such as e.g. collagen, elastin as well as silk protein, substances from the group of glucosaminoglucanes, such as e.g. hyaluronic acid, from the group of carbohydrates, such as e.g. Pentavitin that corresponds in its composition to the carbohydrate mixture of the human corneal layer, and the group of lipids and lipid precursors such as for example ceramides. Further advantageous active agents in the sense of the present invention may further be selected from the group of vitamins, such as e.g. panthenol, niacin, α-tocopherol and its esters, vitamin A as well as vitamin C. Moreover, active agents selected from the group of antioxidants e.g. galates and polyphenols may be used. Urea, hyaluronic acid and Pentavitin are preferred substances.

It is further preferred that substances having skin soothing and regenerative action are employed as active agents, such as e.g. allantoin, evening primrose oil, panthenol, bisabolol and phytosterols.

Advantageous active agents in the sense of the present invention are also plants and plant extracts. These include e.g. algae, aloe, arnica, barber's rash, comfrey, birch, nettle, calendula, oak, ivy, witch-hazel, henna, hop, camomile, ruscus, peppermint, marigold, rosemary, sage, green tea, tea tree, horsetail, thyme and walnut as well as extracts thereof.

The formulations according to the invention may further contain as active agents antimycotics and antiseptics/disinfectants of synthetic or natural origin, for example microsilver.

Further active agents are glucocorticoids, antibiotics, analgetics, antiphlogistics, antirheumatics, antiallergics, antiparasitics, antipruriginostics, antipsoriatics, retinoids, local anaesthetics, therapeutic agents for veins, ceratolytics, hyperaemic substances, coronary therapeutic agents (nitrates/nitro-compounds), antiviral drugs, cytostatics, hormones, agents promoting wound healing, e.g. growth factors, enzyme preparations and insecticides.

In a preferred embodiment the emulsion comprises urea. In another preferred embodiment the emulsion comprises allantoin. In a further preferred embodiment the emulsion comprises urea and allantoin, and optionally further of the above mentioned active agents.

In a further preferred embodiment the emulsion comprises microsilver, preferably in combination with urea and/or allantoin.

### i) Further components

Moreover the emulsion may optionally contain colouring agents, pearlescent pigments, fragrances/perfume, sunscreen filter substances, preservatives, complex formers, antioxidants and repellent agents as well as pH-regulators. However, in a preferred embodiment, the formulations according to the invention are free of substances that may cause skin irritations, in particular free of fragrances/perfume, colouring agents and conventional emulsifiers.

The emulsion may contain, in addition to the components already described above, further natural fats, such as e.g. shea butter, neutral oils, olive oil, squalane, ceramides and moisturing substances as usual in the art.

In a preferred embodiment the emulsion comprises sodium chloride as moisturizer, preferably in an amount of from 0.1 weight-% to 10 weight-%, particularly preferred from 0.5 weight-% to 7 weight-% (based on the total weight of the emulsion without propellant).

The above itemization of individual components of the emulsion is to be understood in the sense that individual exemplified components may also be assignable to several groups, due to their diverse properties.

### k) Propellants

Suitable propellants are e.g. N₂O, propane, butane and i-butane. The complete foam formulation contains for example from 1 to 20 weight-%, from 2 to 18 weight-% or from 5 to 15 weight-%, preferably approximately 10 weight-% of propellant. (Pressure) liquefied propellant is used for charging the emulsion with propellant.

### 3. Methods of manufacture

The foam formulations according to the invention are prepared by providing an emulsion, of the oil-in-water type, and filling said emulsion and optionally charging with a propellant into a suitable container, preferably into a pressurized container. As an alternative to a propellant and a pressurized container, the emulsion may also be filled into another container that is suitable to dispense the emulsion as a foam also in the absence of a propellant. Such systems are known to the person skilled in the art.
a) In a preferred embodiment the method for manufacturing the emulsion comprises the following steps:
   1. Providing a liquid oil phase
   2. Addition of the at least one triterpenoid selected from the group consisting of betulin, betulinic acid, betulinic acid methyl ester, betulin aldehyde, betulonic acid, betulon aldehyde, lupeol, (C1-C6) alkyl esters of the aforementioned acids, or mixtures thereof, to the oil phase,
   3. Providing an aqueous phase
   4. Homogenizing the aqueous phase with the triterpenoid-containing oil phase.

   The addition of the at least one triterpenoid to the oil phase according to step 2 can preferably occur by dispersing the at least one triterpenoid in the oil phase.
b) In such embodiments, wherein the claimed emulsion comprises in addition to the at least one tritcrpcnoid an oil phase, which contains at least one membrane-forming substance forming a lamellar arranged membrane in the foam formulation, in particular the methods of manufacture described in WO 2008/155389 can be used.
   In preferred embodiments, wherein a DMS^{®} base cream or analogous compositions are used as membrane-forming substance, the method just described under a) can be used, comprising the further step:
   5. Stirring the membrane-forming substance into the emulsion obtained in step 4.
c) In such embodiments, wherein the emulsion comprises in addition to the at least one triterpenoid at least one emulsifier-copolymer according to the present invention, for example the methods of manufacture described in the European patent application with the application no. 09 015 330.5 and with the title "Emulsifier-free, polymer-stabilized foam formulations" of the applicant Neubourg Skin Care GmbH & Co. KG in the chapter "Method of manufacture" (pages 35 to 39) can be used. In this respect the at least one triterpenoid is preferably added to the provided oil phase.
   If the at least one emulsifier-copolymer is not used in pre-neutralized form or if otherwise necessary, preferably the pH value of the aqueous phase containing the at least one surface active, ionic polymer is suitably adjusted, before the aqueous phase is added to the oil phase or to the obtained emulsion. The pH adjustment ensures that the at least one surface active, ionic polymer is at least partially neutralized. For example the pH value may be adjusted to 6-7. For this purpose, any suitable base may be used, such as sodium hydroxide, triethanolamine, triisopropanolamine, diethylaminopropylamine, 2-amino-2-methylpropan-1-ol, or trometamol (2-amino-2-hydroxymethyl-propane-1,3-diol). Trometamol is particularly preferred.
d) In such embodiments, wherein the emulsion comprises in addition to the at least one triterpenoid at least one solid emulsifier, for example the methods of manufacture described in WO 2008/138894 can be used. In this respect the at least one triterpenoid is preferably added to the provided oil phase.
e) In such embodiments, wherein the emulsion comprises in addition to the at least one triterpenoid:
   aa) an oil phase comprising at least one membrane-forming substance forming a lamellar arranged membrane in the foam formulation, and at least one emulsifier-copolymer, or
   bb) an oil phase comprising at least one membrane-forming substance forming a lamellar arranged membrane in the foam formulation, and at least one solid emulsifier, or
   cc) at least one emulsifier-copolymer and at least one solid emulsifier, or
   dd) an oil phase comprising at least one membrane-forming substance forming a lamellar arranged membrane in the foam formulation, and at least one emulsifier-copolymer and at least one solid cmulsifier,

it is apparent for the skilled person that combinations of the methods of manufacture described under a) to d) can be used.

### 4. Uses

The foam formulations according to the present invention can be employed for all cosmetic and dermatological purposes (as a medical product or pharmaceutical product). For example, the foam formulations may be employed as skin care agent or skin cleaning agent. Further, they may be used as carriers for active agents and in the medical-dermatological field. In particular the formulations may also be employed as sunscreen, e.g. if they contain solid emulsifiers, such as for example titanium dioxide, which are simultaneously effective UVA and UVB filters.

### 5. Preferred embodiments

The present invention is particularly directed to the following, numbered embodiments:
1. Foam formulation comprising an emulsion, wherein the emulsion is an oil-in-water (O/W) emulsion, wherein the emulsion comprises at least one triterpenoid selected from the group consisting of betulin, betulinic acid, betulinic acid methyl ester, betulin aldehyde, betulonic acid, betulon aldehyde, lupeol, (C1-C6) alkyl esters of the aforementioned acids or mixtures thereof, wherein the foam formulation is present in a pressurized container with a liquefied propellant or without propellant in a container other than a pressurized container that allows for the formation of a foam upon dispensing of the formulation/emulsion.
2. Foam formulation according to embodiment 1, wherein the at least one triterpenoid has a solubility in water of less than 0.5 % (w/w), preferably less than 0.1 % (w/w), particularly preferred less than 0.05 % (w/w), more particularly preferred less than 0.01 % (w/w).
3. Foam formulation according to embodiment 1 or 2, wherein the at least one triterpenoid has a solubility in water of less than 1 µg/ml, preferably less than 0.75 µg/ml, particularly preferred less than 0.5 µg/ml, more particularly preferred less than 0.2 µg/ml.
4. Foam formulation according to any one of the preceding embodiments, wherein the at least one triterpenoid has a solubility in vegetable oil, preferably in refined sunflower oil, of less than 50 mg/ml, preferably less than 20 mg/ml, particularly preferred less than 10 mg/ml, more particularly preferred less than 8 mg/ml or less than 5 mg/ml.
5. Foam formulation according to any one of the preceding embodiments, wherein the at least one triterpenoid is at least partially or substantially present in the emulsion as a particulate solid.
6. Foam formulation according to any one of the preceding embodiments, wherein the emulsion comprises at least one triterpenoid selected from the group consisting of tetracyclic and pentacyclic triterpenoids, or mixtures thereof, preferably comprises at least one pentacyclic triterpenoid.
7. Foam formulation according to any one of the preceding embodiments, wherein the emulsion comprises at least one triterpenoid selected from the group consisting of pentacyclic triterpenoids based on a lupane structure, an ursanc structure, an oleanane structure, a friedelane structure, or mixtures thereof.
8. Foam formulation according to any one of the preceding embodiments, wherein the emulsion comprises at least one triterpenoid selected from the group consisting of betulin, betulinic acid, betulinic acid methyl ester, betulin aldehyde, betulonic acid, betulon aldehyde, lupeol, (C₁-C₆) alkyl esters of the aforementioned acids, derivatives of the aforementioned compounds, or mixtures thereof.
9. Foam formulation according to any one of the preceding embodiments, wherein the emulsion comprises at least one triterpenoid selected from the group consisting ofbetulin, betulinic acid, lupeol, erythrodiol, oleanolic acid, (C₁-C₆) alkyl esters of the aforementioned acids, derivatives of the aforementioned compounds, or mixtures thereof, preferably comprises betulin and/or derivatives thereof.
10. Foam formulation according to any one of the preceding embodiments, wherein the at least one triterpenoid is present in form of at least one triterpenoid-containing plant extract, which is preferably selected from the group consisting of birch cork extracts, extracts from the bark of Betula alba, extracts from the bark of Betula pendula, extracts from the bark of Betula platyphylla, extracts from the resin of the olibanum tree Boswellia, extracts from the resin of the myrrh tree Commiphora, extracts from the bark of the sycamore tree, mistletoe extracts, rosemary extracts, extracts from the roots and/or the leaves of the white ash Fraxinus americana, extracts from the leaves and/or the bark of the mountain ash Sorbus americana, or mixtures thereof.
11. Foam formulation according to any one of the preceding embodiments, wherein the at least one triterpenoid is present in form of at least one triterpenoid-containing plant extract, which is preferably selected from the group consisting of birch cork extracts, extracts from the bark of Betula alba, extracts from the bark of Betula pendula, extracts from the bark of Betula platyphylla or mixtures thereof.
12. Foam formulation according to any one of the preceding embodiments, wherein the at least one plant extract comprises at least 40 weight-% of betulin (based on the total weight of the plant extract).
13. Foam formulation according to any one of the preceding embodiments, wherein the emulsion comprises sodium chloride, preferably in an amount of from 0.1 weight-% to 10 weight-% (based on the total weight of the emulsion without propellant).
14. Foam formulation according to any one of the preceding embodiments, wherein the foam formulation comprises a propellant, preferably a pressure-liquefied propellant.
15. Foam formulation according to any one of the preceding embodiments, wherein the propellant is selected from the group consisting of N₂O, propane, butane, i-butane, and mixtures thereof.
16. Foam formulation according to any one of the preceding embodiments, wherein the foam formulation comprises from 1 to 20 weight-%, preferably from 2 to 18 weight-%, more preferably from 5 to 15 weight-% of propellant.
17. Foam formulation according to any one of the preceding embodiments, wherein the foam formulation is present in a pressurized container.
18. Foam formulation according to any one of the preceding embodiments, wherein the foam formulation is present in form of a foam cream.
19. Foam formulation according to any one of the preceding embodiments, wherein the oil phase comprises at least one membrane-forming substance forming a lamellar arranged membrane in the foam formulation.
20. Foam formulation comprising an emulsion , wherein the emulsion is an oil-in-water (O/W) emulsion, wherein the Emulsion comprises an oil phase and an aqueous phase, wherein the emulsion comprises at least one triterpenoid selected from the group consisting of betulin, betulinic acid, lupeol, (C₁-C₆) alkyl esters of the aforementioned acids, or mixtures thereof, and wherein the oil phase comprises at least one membrane-forming substance forming a lamellar arranged membrane in the emulsion.
21. Foam formulation according to any one of the preceding embodiments, wherein the at least one triterpenoid comprises betulin.
22. Foam formulation according to any one of the preceding embodiments, wherein the emulsion comprises at least 0.05 weight-% of the at least one triterpenoid, preferably at least 0.08 weight-% of the at least one triterpenoid, particularly preferred at least 0.1 weight-% of the at least one triterpenoid, and more particularly preferred at least 0.2 weight-% of the at least one triterpenoid (each based on the total weight of the emulsion without propellant).
23. Foam formulation according to any one of the preceding embodiments, wherein the emulsion comprises from about 0.05 weight-% to about 10 weight-% of the at least one triterpenoid, preferably from about 0.08 weight-% to about 8 weight-% of the at least one triterpenoid, more preferred from about 0.1 weight-% to about 5 weight-% of the at least one triterpenoid and particularly preferred from about 0.2 weight-% to about 3 weight-% of the at least one triterpenoid.
24. Foam formulation according to any one of the preceding embodiments, wherein the emulsion comprises at least 0.05 weight-% of betulin, preferably at least 0.08 weight-% of betulin, particularly preferred at least 0.1 weight-% of betulin and more particularly preferred at least 0.2 weight-% of betulin (each based on the total weight of the emulsion without propellant).
25. Foam formulation according to any one of the preceding embodiments, wherein the emulsion comprises from about 0.05 weight-% to about 10 weight-% of betulin, preferably from about 0.08 weight-% to about 8 weight-% of betulin, particularly preferred from about 0.1 weight-% to about 5 weight-% of betulin and more particularly preferred from about 0.2 weight-% to about 3 weight-% of betulin.
26. Foam formulation according to any one of the embodiments 19 to 25, wherein the at least one membrane-forming substance comprises a lipid, preferably a triglyceride.
27. Foam formulation according to embodiment 26, wherein the triglyceride comprises caprylic acid/capric acid triglyceride.
28. Foam formulation according to embodiment 26 or 27, wherein the lipid further comprises a phospholipid, preferably lecithin.
29. Foam formulation according to embodiment 28, wherein the lecithin comprises a hydrogenated lecithin.
30. Foam formulation according to any one of embodiments 19 to 29, wherein the membrane-forming substance is water insoluble.
31. Foam formulation according to any one of embodiments 19 to 30, wherein the membrane-forming substance has a HLB-value of more than 8, preferably between 9 to 11, more preferably between 9.5 to 10.5.
32. Foam formulation according to any one of the preceding embodiments, wherein the emulsion further comprises at least one surface active, ionic polymer with a molecular weight of more than 5000 g/mol, wherein the ionic polymer is a copolymer comprising as monomer units
   - an ionic monomer (M1), and
   - at least one further monomer.
33. Foam formulation according to embodiment 32, wherein the emulsion comprises from about 0.01 to about 5 weight-%, preferably from about 0.01 to about 1 weight-%, particularly preferred from about 0.01 to about 0.5 weight-%, more particularly preferred from about 0.01 to about 0.1 weight-% of the at least one surface active, ionic polymer (each based on the total weight of the emulsion without propellant).
34. Foam formulation according to embodiment 32 or 33, wherein the ionic monomer (M1) is selected from the group consisting of acrylic acids, methacrylic acids, crotonic acids, maleic acids, fumaric acids, styrene sulfonic acids, vinyl sulfonic acids, vinyl phosphonic acids, allyl sulfonic acids, methallyl sulfonic acids, acrylamido alkylsulfonic acids, which may each be present as free acid, partially or completely neutralized in the form of their salts, preferably the alkali metal salts, alkaline-earth metal salts, ammonium salts or alkanol ammonium salts; or as anhydride, and mixtures thereof; and preferably the ionic monomer (M1) is selected from the group consisting of acrylic acids, methacrylic acids, and acrylamido alkylsulfonic acids.
35. Foam formulation according to any one of embodiments 32 to 34, wherein the ionic monomer (M1) is an acrylamido alkylsulfonic acid having the general formula (1), 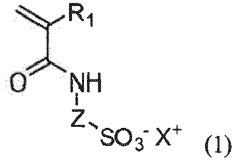 wherein R₁ is selected from the group consisting of hydrogen, methyl or ethyl, Z is a (C₁-C₈)-alkylene, that may be unsubstituted or substituted with one or more (C₁-C₄)-alkyl groups, and X⁺ is selected from the group consisting of H⁺, an alkali metal ion, an alkaline-earth metal ion, an ammonium ion, an alkanol ammonium ion, or mixtures thereof.
36. Foam formulation according to any one of embodiments 32 to 35, wherein the ionic monomer (M1) is 2-acrylamido-2-methylpropane sulfonic acid having the general formula (2) 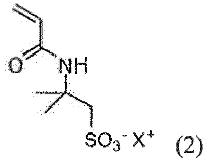 wherein X⁺ is selected from the group consisting of H⁺, an alkali metal ion, an alkaline-earth metal ion, an ammonium ion, an alkanol ammonium ion or mixtures thereof.
37. Foam formulation according to any one of embodiments 32 to 36, wherein the at least one further monomer comprises at least one non-ionic monomer selected from the group consisting of styrenes, chlorostyrenes, di-(C₁-C₃₀)-alkylamino styrenes, vinyl chlorides, isoprenes, vinyl alcohols, vinyl methyl ethers, (C₁-C₃₀)-carboxylic acid vinyl esters, preferably vinyl acetates and vinyl propionates; acrylic acid esters, methacrylic acid esters, maleic acid esters, fumaric acid esters, crotonic acid esters; in particular linear and branched (C₁-C₃₀)-alkyl esters of acrylic acid, methacrylic acid, maleic acid, fumaric acid and crotonic acid; linear and branched (C₁-C₃₀)-hydroxyalkyl esters of acrylic acid, methacrylic acid, maleic acid, fumaric acid and crotonic acid; ethoxylated (C₁-C₃₀)-alkyl esters of acrylic acid, methacrylic acid, maleic acid, fumaric acid and crotonic acid having from 1 to 40 ethylene oxide units; acrylamides, in particular N,N-di-(C₁-C₃₀)-alkyl acrylamides, methacrylamides, in particular N,N-di-(C₁-C₃₀)-alkyl methacrylamides, cyclic and linear N-vinyl carboxylic acid amides having a carbon chain of 2 to 9 carbon atoms, preferably N-vinylpyrrolidone; and mixtures thereof.
38. Foam formulation according to any one of embodiments 32 to 37, wherein the at least one further monomer comprises at least one ionic monomer selected from the group consisting of acrylic acids, methacrylic acids, crotonic acids, maleic acids, fumaric acids, styrene sulfonic acids, vinyl sulfonic acids, vinyl phosphonic acids, allyl sulfonic acids, methallyl sulfonic acids, acrylamido alkylsulfonic acids, which may each be present as free acid, partially or completely neutralized in the form of their salts, preferably the alkali metal salts, alkaline-earth metal salts or ammonium salts; or as anhydride, and mixtures thereof.
39. Foam formulation according to any one of embodiments 32 to 38, wherein the at least one surface active, ionic polymer is selected from the group consisting of acryloyldimethyltaurate/vinylpyrrolidone copolymer, sodium acrylate/ acryloyldimethyltaurate/ dimethylacrylamide crosspolymer, hydroxyethyl acrylate/ sodium acryloyldimethyltaurate copolymer, sodium acrylate/ sodium acryloyldimethyltaurate copolymer, and mixtures thereof.
40. Foam formulation according to any one of embodiments 32 to 39, wherein the at least one surface active ionic polymer is acryloyldimethyltaurate/ vinylpyrrolidone copolymer, particularly preferred ammonium acryloyldimethyltaurate/ vinylpyrrolidone copolymer.
41. Foam formulation according to any one of the preceding embodiments, wherein the emulsion further comprises at least one solid emulsifier.
42. Foam formulation according to any one of the preceding embodiments, wherein the emulsion further comprises at least one solid emulsifier, selected from the group consisting of titanium dioxide, silicon dioxide, Fe₂O₃, zinc oxide, veegum, bentonite and ethyl cellulose, aluminium oxide, calcium carbonate, coal, magnesium oxide, magnesium trisilicate, crystalline fatty acids, crystalline fatty acid esters, crystalline fatty alcohols, polymer lattices such as polystyrenes or polymethacrylates, and polymer-pseudo lattices or mixtures thereof.
43. Foam formulation according to embodiment 41 or 42, wherein the emulsion comprises from 0.5 to 7 weight-%, preferably from 0.5 to 5 weight-%, particularly preferred from 0.5 to 3 weight-% of the at least one solid emulsifier.
44. Foam formulation according to any one of the preceding embodiments, wherein the emulsion comprises at least one active agent.
45. Foam formulation according to any one of the preceding embodiments, wherein the emulsion is an oil-in-water emulsion.
46. Foam formulation according to any one of the preceding embodiments, wherein the emulsion is a substantially emulsifier-free emulsion.
47. Foam formulation according to any one of the preceding embodiments, wherein the emulsion is an emulsifier-free emulsion.
48. Use of an O/W emulsion comprising an oil phase and an aqueous phase, wherein the emulsion comprises at least one triterpenoid, for the manufacture of a foam formulation.
49. Use of the claimed O/W emulsion, comprising an oil phase and an aqueous phase, wherein the emulsion comprises at least one triterpenoid, for the manufacture of a foam formulation according to any one of the preceding embodiments.
50. Use according to embodiment 48 or 49, wherein the oil phase of the emulsion comprises at least one membrane-forming substance forming a lamellar arranged membrane in the foam formulation.
51. Use according to embodiments 48 to 50, wherein the emulsion is substantially emulsifier-free.
52. Use of at least one triterpenoid for the stabilization of foam formulations comprising an emulsion, preferably according to any one of the preceding embodiments 1 to 47.
53. Use according to embodiment 52, wherein the oil phase of the emulsion comprises at least one membrane-forming substance forming a lamellar arranged membrane in the foam formulation.
54. Use of a foam formulation according to any one of the preceding embodiments as carrier for an active agent, as skin care agent, as skin cleaning agent, as sunscreen, or for the manufacture of a cosmetic, a medical product or a medicament.
55. Use of an emulsion according to any one of the preceding embodiments as carrier for an active agent, as skin care agent, as skin cleaning agent, as sunscreen, or for the manufacture of a cosmetic, a medical product or a medicament.

### 6. Examples

Foam creams having the following composition of the emulsion were prepared:

| **Ingredient** | **INCI- or common name** | **Percentage [weight-%]** | | |
|---|---|---|---|---|
| | | **Exampe 1** | **Example 2** | **Example 3** |
| **Phase A:** | | | | |
| Cetiol V | Decyl oleate | 5.00 | 5.00 | 5.00 |
| Eutanol G | Octyldodecanol | 5.00 | 5.00 | 5.00 |
| Edenor C18 98/100 | Stearic acid | 2.50 | 2.50 | 2.50 |
| Keltrol CG | Xanthan Gum | 0.02 | 0.02 | 0.02 |
| Arctic Birch Bark Extract | Birch bark extract | 0.30 | -- | 0.30 |
| Ritalab White Birch Bark Extract P | Birch bark extract | -- | 0.30 | -- |
| | | | | |

| **Phase B:** | | | | |
|---|---|---|---|---|
| demineralized water | | 63.03 | 63.03 | 61.93 |
| Sodium chloride | | 5.50 | 5.50 | 5.50 |
| Urea | | 5.50 | 5.50 | 5.50 |
| Glycerol 86% | | 5.00 | 5.00 | 5.00 |
| Allantoin powder | | 0.10 | 0.10 | 0.10 |
| Phospholipon H80 | hydrogenated phosphatidylcholine | 3.00 | 3.00 | 3.00 |
| Cosmedia SP | Sodium polyacrylate | 0.03 | 0.03 | 0.03 |
| Aristoflex AVC | Ammonium acryloyldimethyltaurate VP-copolymer | 0.02 | 0.02 | 0.02 |
| | | | | |

| **"Phase C":** | | | | |
|---|---|---|---|---|
| DMS¹ | | 5.00 | 5.00 | 5.00 |
| Evening primrose oil | | -- | -- | 1.00 |
| Microsilver | | -- | -- | 0.10 |
| | | | | |
| **Total** | | **100.00** | **100.00** | **100.00** |

| | | | | |
|---|---|---|---|---|
| ¹ The DMS-basis employed is Probiol from the company KUHS having the following composition: water, hydrogenated lecithin, caprylic acid/capric acid triglyceride, pentylene glycol, butyrospermum parkii butter, glycerol, squalane, ceramide 3. | | | | |

### Manufacture of the emulsion:

Cetiol V, Eutanol G and Edenor C18 98/100 are heated to 60 °C in the preparation vessel while stirring at 2000 rpm. Subsequently Keltrol CG and the birch bark extract are added. The mixture is heated to 70 °C and stirring is continued for about 20 minutes.

The demineralized water is provided and sodium chloride, urea, glycerol and allantoin are added. The mixture is heated to 60 °C while stirring at 2000 rpm. At this temperature, phospholipon H 80, Cosmedia SP and Aristoflex AVC are slowly added. The mixture is heated to 70 °C and stirring is continued for about 20 to 30 minutes at 2000 rpm, until a homogeneous, thickened liquid is formed.

The aqueous phase (B) is admixed to the oil phase (A) at approximately 70 °C. At this temperature stirring is continued at 2000 rpm for about 20 minutes. Subsequently the mixture is allowed to cool down while stirring. At a temperature of 35 °C and 2000 rpm the DMS-concentrate is homogeneously stirred into the mixture.

In example 3, the microsilver is stirred into the evening primrose oil. This mixture is also stirred into the emulsion at 35 °C.

### Manufacture of the foam formulation:

86.5 g of the emulsion are each filled into aluminium monoblock cans and are charged with 9.5 g of propellant (propane-butane-mixture).

### Foam formation:

A foam is formed upon dispensing the foam formulations of examples 1 to 3 from the pressurized container by means of a suitable valve having a foam applicator attached. The structure of the foam is maintained for a time period that is sufficient to uniformly distribute the foam onto the skin.

## Claims

1. Foam formulation comprising an emulsion, comprising an oil phase and an aqueous phase, wherein the emulsion is an oil-in-water emulsion, wherein the emulsion comprises at least one triterpenoid selected from the group consisting of betulin, betulinic acid, betulinic acid methyl ester, betulin aldehyde, betulonic acid, betulon aldehyde, lupeol, (C₁-C₆) alkyl esters of the aforementioned acids, or mixtures thereof, wherein the foam formulation is present in a pressurized container with a liquefied propellant or without propellant in a container other than a pressurized container that allows for the formation of a foam upon dispensing of the formulation/emulsion.

2. Foam formulation according to claim 1, wherein from 10 weight-% to 60 weight-% or from 60 weight-% to 100 weight-% of the utilized amount of the at least one triterpenoid are present in the emulsion as a particulate solid.

3. Foam formulation according to any one of the preceding claims, wherein the foam formulation comprises from 1 to 20 weight-% of propellant.

4. Foam formulation according to any one of the preceding claims, wherein the oil phase comprises at least one membrane-forming substance forming a lamellar arranged membrane in the foam formulation, wherein the at least one membrane-forming substance comprises a lipid, wherein the lipid comprises a phospholipid.

5. Foam formulation according to any one of the preceding claims, wherein the at least one triterpenoid comprises betulin.

6. Foam formulation according to any one of the preceding claims, wherein the emulsion comprises at least 0.05 weight-% of the at least one triterpenoid (based on the total weight of the emulsion without propellant).

7. Foam formulation according to any one of the preceding claims, wherein the emulsion comprises from about 0.05 weight-% to about 10 weight-% of the at least one triterpenoid.

8. Foam formulation according to any one of the preceding claims, wherein the emulsion comprises from about 0.05 weight-% to about 10 weight-% of betulin.

9. Foam formulation according to any one of claims 4 to 8, wherein the at least one membrane-forming substance comprises a phospholipid and a triglyceride.

10. Foam formulation according to claim 9, wherein the triglyceride comprises caprylic acid/capric acid triglyceride.

11. Foam formulation according to claim 9 or 10, wherein the phospholipid comprises lecithin.

12. Foam formulation according to any one of claims 4 to 11, wherein the membrane-forming substance has a HLB-value of between 9 to 11.

13. Foam formulation according to any one of the preceding claims, wherein the emulsion further comprises at least one surface active, ionic polymer with a molecular weight of more than 5000 g/mol, wherein the ionic polymer is a copolymer comprising as monomer units
- an ionic monomer (M1), and
- at least one further monomer.

14. Foam formulation according to claim 13, wherein the emulsion comprises from about 0.01 to about 5 weight-% of the at least one surface active, ionic polymer (based on the total weight of the emulsion without propellant).

15. Foam formulation according to claim 13 or 14, wherein the ionic monomer (M1) is selected from the group consisting of acrylic acids, methacrylic acids, crotonic acids, maleic acids, fumaric acids, styrene sulfonic acids, vinyl sulfonic acids, vinyl phosphonic acids, allyl sulfonic acids, methallyl sulfonic acids, acrylamido alkylsulfonic acids, which may each be present as free acid, partially or completely neutralized in the form of their salts; or as anhydride, and mixtures thereof.

16. Foam formulation according to any one of claims 13 to 15, wherein the ionic monomer (M1) is an acrylamido alkylsulfonic acid having the general formula (1), 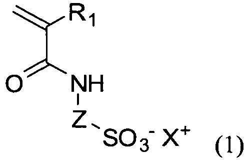 wherein R₁ is selected from the group consisting of hydrogen, methyl or ethyl, Z is a (C₁-C₈)-alkylene, that may be unsubstituted or substituted with one or more (C₁-C₄)-alkyl groups, and X⁺ is selected from the group consisting of H⁺, an alkali metal ion, an alkaline-earth metal ion, an ammonium ion, an alkanol ammonium ion, or mixtures thereof.

17. Foam formulation according to any one of the preceding claims, wherein the emulsion further comprises at least one solid emulsifier.

18. Foam formulation according to any one of the preceding claims, wherein the emulsion contains less than 1.0 weight-% of conventional emulsifiers, wherein conventional emulsifiers are amphiphilic substances having a molecular weight < 5000 g/mol, which can form micelles.

19. Use of an emulsion, comprising an oil phase and an aqueous phase, wherein the emulsion is an oil-in-water emulsion, wherein the emulsion comprises at least one triterpenoid, for the manufacture of a foam formulation, wherein the at least one triterpenoid is selected from the group consisting of betulin, betulinic acid, betulinic acid methyl ester, betulin aldehyde, betulonic acid, betulon aldehyde, lupeol, (C₁-C₆) alkyl esters of the aforementioned acids, or mixtures thereof, wherein the foam formulation is present in a pressurized container with a liquefied propellant or without propellant in a container other than a pressurized container that allows for the formation of a foam upon dispensing of the formulation/emulsion.

20. Use of at least one triterpenoid for the stabilization of foam formulations comprising an emulsion, the emulsion comprising an oil phase and an aqueous phase, wherein the emulsion is an oil-in-water emulsion, wherein the at least one triterpenoid is selected from the group consisting of betulin, betulinic acid, betulinic acid methyl ester, betulin aldehyde, betulonic acid, betulon aldehyde, lupeol, (C₁-C₆) alkyl esters of the aforementioned acids, or mixtures thereof, wherein the foam formulations are present in a pressurized container with a liquefied propellant or without propellant in a container other than a pressurized container that allows for the formation of a foam upon dispensing of the formulation/emulsion.

21. Use according to claim 19 or 20, wherein the foam formulation is a foam formulation according to any one of claims 1 to 18.

22. Use of a foam formulation according to any one of claims 1 to 18, as carrier for an active agent, as skin care agent, as skin cleaning agent, as sunscreen, or for the manufacture of a cosmetic, a medical product or a medicament.

## Patentansprüche

1. Schaumformulierung, welche eine Emulsion umfasst, die eine Ölphase und eine Wasserphase aufweist, wobei die Emulsion eine Öl-in-Wasser-Emulsion ist, wobei die Emulsion mindestens ein Triterpenoid aufweist, ausgewählt aus der Gruppe bestehend aus Betulin, Betulinsäure, Betulinsäure-Methylester, Betulinaldehyd, Betulonsäure, Betulonaldehyd, Lupeol, (C₁-C₆)-Alkylestern der vorstehenden Säuren oder Mischungen davon, wobei die Schaumformulierung in einem druckbeaufschlagten Behälter mit einem verflüssigten Treibmittel oder ohne Treibmittel in einem anderen Behälter als einem druckbeaufschlagten Behälter vorliegt, der bei Abgabe der Formulierung/Emulsion die Bildung eines Schaums erlaubt.

2. Schaumformulierung nach Anspruch 1, wobei von 10 Gewichts-% bis 60 Gewichts-% oder von 60 Gewichts-% bis 100 Gewichts-% der verwendeten Menge des mindestens einen Triterpenoids in der Emulsion als Partikelfeststoff vorliegen.

3. Schaumformulierung nach einem der vorhergehenden Ansprüche, wobei die Schaumformulierung von 1 bis 20 Gewichts-% Treibmittel umfasst.

4. Schaumformulierung nach einem der vorhergehenden Ansprüche, wobei die Ölphase mindestens eine membranbildende Substanz aufweist, die eine lamellenartig ausgebildete Membran in der Schaumformulierung bildet, wobei die mindestens eine membranbildende Substanz ein Lipid aufweist, wobei das Lipid ein Phospholipid umfasst.

5. Schaumformulierung nach einem der vorhergehenden Ansprüche, wobei das mindestens eine Triterpenoid Betulin umfasst.

6. Schaumformulierung nach einem der vorhergehenden Ansprüche, wobei die Emulsion mindestens 0,05 Gewichts-% des mindestens einen Triterpenoids aufweist (bezogen auf das Gesamtgewicht der Emulsion ohne Treibmittel).

7. Schaumformulierung nach einem der vorhergehenden Ansprüche, wobei die Emulsion von etwa 0,05 Gewichts-% bis etwa 10 Gewichts-% des mindestens einen Triterpenoids aufweist.

8. Schaumformulierung nach einem der vorhergehenden Ansprüche, wobei die Emulsion von etwa 0,05 Gewichts-% bis etwa 10 Gewichts-% Betulin aufweist.

9. Schaumformulierung nach einem der Ansprüche 4 bis 8, wobei die mindestens eine membranbildende Substanz ein Phospholipid und ein Triglycerin aufweist.

10. Schaumformulierung nach Anspruch 9, wobei das Triglycerid Caprylsäure-/Caprinsäure-Triglycerid umfasst.

11. Schaumformulierung nach Anspruch 9 oder 10, wobei das Phospholipid Lecithin umfasst.

12. Schaumformulierung nach einem der Ansprüche 4 bis 11, wobei die membranbildende Substanz einen HLB-Wert zwischen 9 und 11 aufweist.

13. Schaumformulierung nach einem der vorhergehenden Ansprüche, wobei die Emulsion ferner mindestens ein oberflächenaktives ionisches Polymer mit einem Molekulargewicht größer als 5000 g/mol aufweist, wobei das ionische Polymer ein Copolymer ist, umfassend als Monomereinheiten
- ein ionisches Monomer (M1) und
- mindestens ein weiteres Monomer.

14. Schaumformulierung nach Anspruch 13, wobei die Emulsion von etwa 0,01 bis etwa 5 Gewichts-% des mindestens einen oberflächenaktiven ionischen Polymers aufweist (bezogen auf das Gesamtgewicht der Emulsion ohne Treibmittel).

15. Schaumformulierung oder Emulsion nach Anspruch 13 oder 14, wobei das ionische Monomer (M1) ausgewählt ist aus der Gruppe bestehend aus Acrylsäuren, Methacrylsäuren, Crotonsäuren, Maleinsäuren, Fumarinsäuren, Styrolsulfonsäuren, Vinylsulfonsäuren, Vinylphosphonsäuren, Allylsulfonsäuren, Methallylsulfonsäuren, Acrylamidoalkylsulfonsäuren, die jeweils als freie Säure, teilweise oder vollständig neutralisiert in Form ihrer Salz oder als Anhydrid und Mischungen davon vorliegen können.

16. Schaumformulierung nach einem der Ansprüche 13 bis 15, wobei das ionische Monomer (M1) eine Acrylamidoalkylsulfonsäure mit der allgemeinen Formel (1) ist, 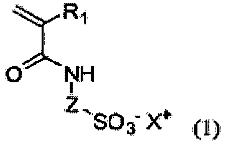 wobei R₁ ausgewählt ist aus der Gruppe bestehend aus Wasserstoff, Methyl oder Ethyl, Z ein (C₁-C₈)-Alkylen ist, das unsubstituiert oder mit einer oder mehreren (C₁-C₄)-Alkylgruppen substituiert sein kann, und X⁺ ausgewählt ist aus der Gruppe bestehend aus H⁺, einem Alkalimetallion, einem Erdalkalimetallion, einem Ammoniumion, einem Alkanolammoniumion oder Mischungen davon.

17. Schaumformulierung nach einem der vorhergehenden Ansprüche, wobei die Emulsion ferner mindestens einen festen Emulgator aufweist.

18. Schaumformulierung nach einem der vorhergehenden Ansprüche, wobei die Emulsion weniger als 1,0 Gew.-% von konventionellen Emulgatoren enthält, wobei die konventionellen Emulgatoren amphiphile Substanzen mit einem Molekulargewicht <5000 g/mol sind, die Mizellen bilden können.

19. Verwendung einer Emulsion, die eine Ölphase und eine Wasserphase aufweist, wobei die Emulsion eine Öl-in-Wasser-Emulsion ist, wobei die Emulsion mindestens ein Triterpenoid aufweist, für die Herstellung einer Schaumformulierung, wobei das mindestens eine Triterpenoid ausgewählt ist aus der Gruppe bestehend aus Betulin, Betulinsäure, Betulinsäure-Methylester, Betulinaldehyd, Betulonsäure, Betulonaldehyd, (C₁-C₆)-Alkylestern der vorstehenden Säuren oder Mischungen davon, wobei die Schaumformulierung in einem druckbeaufschlagten Behälter mit einem verflüssigten Treibmittel oder ohne Treibmittel in einem anderen Behälter als einem druckbeaufschlagten Behälter vorliegt, der bei Abgabe der Formulierung/Emulsion die Bildung eines Schaums erlaubt.

20. Verwendung mindestens eines Triterpenoids für die Stabilisierung von Schaumformulierungen, umfassend eine Emulsion, wobei die Emulsion eine Ölphase und eine Wasserphase aufweist, wobei die Emulsion eine Öl-in-Wasser-Emulsion ist, wobei das mindestens eine Triterpenoid ausgewählt ist aus der Gruppe bestehend aus Betulin, Betulinsäure, Betulinsäure-Methylester, Betulinaldehyd, Betulonsäure, Betulonaldehyd, Lupeol, (C1-C6)-Alkylestern der vorstehenden Säuren oder Mischungen davon, wobei die Schaumformulierungen in einem druckbeaufschlagten Behälter mit einem verflüssigten Treibmittel oder ohne Treibmittel in einem anderen Behälter als einem druckbeaufschlagten Behälter vorliegen, der bei Abgabe der Formulierung/Emulsion die Bildung eines Schaums erlaubt.

21. Verwendung nach Anspruch 19 oder 20, wobei die Schaumformulierung eine Schaumformulierung nach einem der Ansprüche 1 bis 18 ist.

22. Verwendung einer Schaumformulierung nach einem der Ansprüche 1 bis 18 als Träger für einen Wirkstoff, als Hautpflegemittel, als Hautreinigungsmittel, als Sonnenschutz oder für die Herstellung eines kosmetischen Mittels, eines medizinischen Produkts oder eines Medikaments.

## Revendications

1. Formulation de mousse comprenant une émulsion, comprenant une phase d'huile et une phase aqueuse, dans laquelle l'émulsion est une émulsion de type huile-dans-eau, dans laquelle l'émulsion comprend au moins un triterpénoïde choisi à partir du groupe consistant en bétuline, acide bétulinique, ester méthylique d'acide bétulinique, aldéhyde bétulinique, acide bétulonique, aldéhyde bétulonique, lupéol, esters d'alkyle en C₁ à C₆ des acides précités ou de mélanges de ceux-ci, dans laquelle la formulation de mousse est présente dans un récipient pressurisé avec un propulseur liquéfié ou sans propulseur dans un récipient autre qu'un récipient pressurisé qui permet la formation d'une mousse lors de la délivrance de la formulation/émulsion.

2. Formulation de mousse selon la revendication 1, dans laquelle entre 10 % en poids et 60 % en poids ou entre 60 % en poids et 100 % en poids de la quantité utilisée de l'au moins un triterpénoïde sont présents dans l'émulsion comme solide particulaire.

3. Formulation de mousse selon une des revendications précédentes, dans laquelle la formulation de mousse comprend entre 1 et 20 % en poids de propulseur.

4. Formulation de mousse selon une des revendications précédentes, dans laquelle la phase d'huile comprend au moins une substance formant membrane, formant une membrane disposée de façon lamellaire dans la formulation de mousse, dans laquelle au moins une substance formant membrane comprend un lipide, le lipide comprenant un phospholipide.

5. Formulation de mousse selon une des revendications précédentes, dans laquelle l'au moins un triterpénoïde comprend de la bétuline.

6. Formulation de mousse selon une des revendications précédentes, dans laquelle l'émulsion comprend au moins 0,05 % en poids de l'au moins un triterpénoïde (sur la base du poids total de l'émulsion sans propulseur).

7. Formulation de mousse selon une des revendications précédentes, dans laquelle l'émulsion comprend entre environ 0,05 % en poids et environ 10 % en poids de l'au moins un triterpénoïde.

8. Formulation de mousse selon une des revendications précédentes, dans laquelle l'émulsion comprend entre environ 0,05 % en poids et environ 10 % en poids de la bétuline.

9. Formulation de mousse selon une des revendications 4 à 8, dans laquelle l'au moins une substance formant membrane comprend un phospholipide et un triglycéride.

10. Formulation de mousse selon la revendication 9, dans laquelle le triglycéride comprend un triglycéride d'acide caprylique/caprique.

11. Formulation de mousse selon la revendication 9 ou 10, dans laquelle le phospholipide comprend de la lécithine.

12. Formulation de mousse selon une des revendications 4 à 11, dans laquelle la substance formant membrane a une valeur HLB entre 9 et 11.

13. Formulation de mousse selon une des revendications précédentes, dans laquelle l'émulsion en outre comprend au moins un polymère ionique tensioactif avec un poids moléculaire supérieur à 5000 g/mol, dans laquelle le polymère ionique est un copolymère comprenant comme des unités monomériques
- un monomère ionique (M1) et
- au moins un monomère additionnel.

14. Formulation de mousse selon la revendication 13, dans laquelle l'émulsion comprend entre environ 0,01 et environ 5 % en poids de l'au moins un polymère ionique tensioactif (sur la base du poids total de l'émulsion sans propulseur).

15. Formulation de mousse ou émulsion selon la revendication 13 ou 14, dans laquelle le monomère ionique (M1) est choisi à partir du groupe consistant en des acides acryliques, acides méthacryliques, acides crotoniques, acides maléiques, acides fumariques, acides styrène-sulfoniques, acides vinyle-sulfoniques, acides vinyle-phosphoniques, acides allyle-sulfoniques, acides méthallyle-sulfoniques, acides acrylamidoalkyle-sulfoniques, chacun desquels pouvant être présenté comme acide libre, partiellement ou complètement neutralisé sous la forme de leurs sels; ou comme anhydride et des mélanges de ceux-ci.

16. Formulation de mousse selon une des revendications 13 à 15, dans laquelle le monomère ionique (M1) est un acide acrylamidoalkyle-sulfonique avec la formule générale (1), 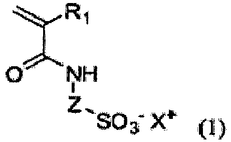 dans laquelle R₁ est choisi à partir du groupe consistant en hydrogène, méthyle ou éthyle, Z est un alkylène en C₁ à C₈ qui peut être substitué ou non par un ou plusieurs groupes alkyles en C₁ à C₄, et X⁺ est choisi à partir du groupe consistant en H+, un ion d'un métal alcalin, un ion d'un métal alcalino-terreux, un ion d'ammonium, un ion d'alcanol-ammonium ou des mélanges de ceux-ci.

17. Formulation de mousse selon une des revendications précédentes, dans laquelle l'émulsion en outre comprend au moins un émulsifiant solide.

18. Formulation de mousse selon une des revendications précédentes, dans laquelle l'émulsion contient moins de 1,0 % en poids d'émulsifiants conventionnels, dans laquelle les émulsifiants conventionnels sont des substances amphiphiliques avec un poids moléculaire <5000 g/mol, qui peuvent former des micelles.

19. Utilisation d'une émulsion, comprenant une phase d'huile et une phase aqueuse, dans laquelle l'émulsion est une émulsion de type huile-dans-eau, dans laquelle l'émulsion comprend au moins un triterpénoïde, pour la fabrication d'une formulation de mousse, dans laquelle l'au moins un triterpénoïde est choisi à partir du groupe consistant en bétuline, acide bétulinique, acide bétulinique ester méthylique, aldéhyde bétulinique, acide bétulonique, aldéhyde bétulonique, esters d'alkyle en C₁ à C₆ des acides précités ou des mélanges de ceux-ci, dans laquelle la formulation de mousse est présente dans un récipient pressurisé avec un propulseur liquéfié ou sans propulseur dans un récipient autre qu'un récipient pressurisé qui permet la formation d'une mousse lors de la délivrance de la formulation/émulsion.

20. Utilisation d'au moins un triterpénoïde pour la stabilisation de formulations de mousse comprenant une émulsion, l'émulsion comprenant une phase d'huile et une phase aqueuse, dans laquelle l'émulsion est une émulsion de type huile-dans-eau, dans laquelle l'au moins un triterpénoïde est choisi à partir du groupe consistant en bétuline, acide bétulinique, acide bétulinique ester méthylique, aldéhyde bétulinique, acide bétulonique, aldéhyde bétulonique, lupéol, esters d'alkyle en C₁ à C₆ des acides précités ou des mélanges de ceux-ci, dans laquelle la formulation de mousse est présente dans un récipient pressurisé avec un propulseur liquéfié ou sans propulseur dans un récipient autre qu'un récipient pressurisé qui permet la formation d'une mousse lors de la délivrance de la formulation/émulsion.

21. Utilisation selon la revendication 19 ou 20, dans laquelle la formulation de mousse est une formulation de mousse selon une des revendications 1 à 18.

22. Utilisation d'une formulation de mousse selon une des revendications 1 à 18, comme excipient pour un agent actif, comme agent de soin pour la peau, comme filtre solaire, ou pour la fabrication d'un produit cosmétique, d'un produit médical ou d'un médicament.
